# EUROPEAN PATENT APPLICATION

(11) **EP 1 324 044 A1**
(43) Date of publication of application: **02.07.2003**
(21) Application number: 01403359.1
(22) Date of filing: 24.12.2001
(51) Int. Cl.: G01N 33/574, G01N 33/50

(54) **FADD proteins, phosphorylated p38-MAPK and FasL as tumour markers**

(71) Applicant: INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75794 Paris Cedex 16 (FR)
(72) Inventor: Chioccia, Gilles, 95270 Luzarches (FR); Tourneur, Léa, 75012 Paris (FR); Feunteun, Jean, 75005 Paris (FR); Michiels, Francine, 94240 L'Hay les Roses (FR)
(74) Representative: Nargolwalla, Cyra

(57) **Abstract**

The application relates to the use of at least one protein selected from the group consisting of FADD proteins and phosphorylated p38-MAPK, as a marker for absence of *in vivo* tumour. This use may further be complemented by the use of the FasL protein as a marker for presence of *in vivo* tumour. The (respective) content(s) in FADD proteins and phosphorylated p38-MAPK indeed decrease, sometimes up to zero, with tumour development, while FasL expression is gained.

SEQUENCE LISTING

<110> I.N.S.E.R.M. (Institut National de la Santé et de la Recherche Médicale)
C.N.R.S (Centre National de la Recherche Scientifique)

<120> New tumour markers and biological applications thereof

<130> FP-CHIOCCHIA

<140> EP01403359.1
<141> 2001-12-24

<160> 26

<170> PatentIn version 3.1

<210> 1
<211> 22
<212> DNA
<213> Artificial Sequence

<220>
<223> primer
<400> 1
cagcagtgcc acttcatctt gg 22

<210> 2
<211> 22
<212> DNA
<213> Artificial Sequence

<220>
<223> primer
<400> 2
ttcactccag agatcagagc gg 22

<210>  3

<211>  20

<212>  DNA

<213>  Artificial Sequence



<220>

<223>  primer

<400>  3
cttctactgc gattctcctg                                                    20

<210>  4

<211>  20

<212>  DNA

<213>  Artificial Sequence



<220>

<223>  primer

<400>  4
cttgccctcc ttgatgttat                                                    20

<210>  5

<211>  20

<212>  DNA

<213>  Artificial Sequence



<220>

<223>  primer

<400>  5
tggatgggat tgaggagaag                                                    20

<210>  6

<211>  20

<212>  DNA

<213>  Artificial Sequence



<220>

<223>  primer

<400> 6
aaaccacagt cctcacaggg 20

<210> 7

<211> 18

<212> DNA

<213> Artificial Sequence

<220>

<223> primer

<400> 7
ccaacctacc ccccaatc 18

<210> 8

<211> 20

<212> DNA

<213> Artificial Sequence

<220>

<223> primer

<400> 8
gcctcagagg cagtgttttc 20

<210> 9

<211> 21

<212> DNA

<213> Artificial Sequence

<220>

<223> primer

<400> 9
caggagcaag aggtcattct g 21

<210> 10

<211> 22

<212> DNA

<213> Artificial Sequence

<220>

<223>  primer

<400>  10
tgttatcaaa gatggcttgg tg                                              22

<210>  11

<211>  21

<212>  DNA

<213>  Artificial Sequence

<220>

<223>  primer

<400>  11
gctgacctca gatgtgagag g                                               21

<210>  12

<211>  22

<212>  DNA

<213>  Artificial Sequence

<220>

<223>  primer

<400>  12
tcccattgaa gatgtacaca gc                                              22

<210>  13

<211>  20

<212>  DNA

<213>  Artificial Sequence

<220>

<223>  primer

<400>  13
tacagagtga ggcggtttga                                                 20

<210>  14

<211>  20

<212>  DNA

<213>  Artificial Sequence

<220>

<223>  primer

<400>  14
agcgaagcct ggagagtatt                                                    20

<210>  15

<211>  20

<212>  DNA

<213>  Artificial Sequence

<220>

<223>  primer

<400>  15
cgagaagaag ggagaatcac                                                    20

<210>  16

<211>  20

<212>  DNA

<213>  Artificial Sequence

<220>

<223>  primer

<400>  16
cataaggcaa ccacaccatc                                                    20

<210>  17

<211>  20

<212>  DNA

<213>  Artificial Sequence

<220>

<223>  primer

<400>  17
agcccagagt atgttccaga                                                    20

<210>  18

<211>  20

<212>  DNA

<213>  Artificial Sequence

<220>

<223>  primer

<400>  18
ctcaaactca tcgctcatcc                                                    20

<210>  19

<211>  20

<212>  DNA

<213>  Artificial Sequence

<220>

<223>  primer

<400>  19
tgctacaggg tttcatccag                                                    20

<210>  20

<211>  20

<212>  DNA

<213>  Artificial Sequence

<220>

<223>  primer

<400>  20
caaagtagaa gagggcaacc                                                    20

<210>  21

<211>  25

<212>  DNA

<213>  Artificial Sequence

<220>

<223>  primer

<400>  21
tggaatcctg tggcatccat gaaac                                              25

<210>  22

<211>  25

<212>  DNA

<213>  Artificial Sequence

<220>

<223>  primer

<400>  22
taaaacgcag ctcagtaaca gtccg                                                25

<210>  23

<211>  208

<212>  PRT

<213>  Homo sapiens

<400>  23

Met Asp Pro Phe Leu Val Leu Leu His Ser Val Ser Ser Ser Leu Ser
1               5                   10                  15

Ser Ser Glu Leu Thr Glu Leu Lys Phe Leu Cys Leu Gly Arg Val Gly
            20                  25                  30

Lys Arg Lys Leu Glu Arg Val Gln Ser Gly Leu Asp Leu Phe Ser Met
        35                  40                  45

Leu Leu Glu Gln Asn Asp Leu Glu Pro Gly His Thr Glu Leu Leu Arg
    50                  55                  60

Glu Leu Leu Ala Ser Leu Arg Arg His Asp Leu Leu Arg Arg Val Asp
65                  70                  75                  80

Asp Phe Glu Ala Gly Ala Ala Ala Gly Ala Ala Pro Gly Glu Glu Asp
                85                  90                  95

Leu Cys Ala Ala Phe Asn Val Ile Cys Asp Asn Val Gly Lys Asp Trp
            100                 105                 110

Arg Arg Leu Ala Arg Gln Leu Lys Val Ser Asp Thr Lys Ile Asp Ser
        115                 120                 125

Ile Glu Asp Arg Tyr Pro Arg Asn Leu Thr Glu Arg Val Arg Glu Ser
    130                 135                 140

Leu Arg Ile Trp Lys Asn Thr Glu Lys Glu Asn Ala Thr Val Ala His
145                 150                 155                 160

Leu Val Gly Ala Leu Arg Ser Cys Gln Met Asn Leu Val Ala Asp Leu
                165                 170                 175

Val Gln Glu Val Gln Gln Ala Arg Asp Leu Gln Asn Arg Ser Gly Ala
            180                 185                 190

Met Ser Pro Met Ser Trp Asn Ser Asp Ala Ser Thr Ser Glu Ala Ser
        195                 200                 205

<210>  24

<211>  205

<212>  PRT

<213>  Mus musculus

<400>  24

Met Asp Pro Phe Leu Val Leu Leu His Ser Leu Ser Gly Ser Leu Ser
1               5                   10                  15

Gly Asn Asp Leu Met Glu Leu Lys Phe Leu Cys Arg Glu Arg Val Ser
            20                  25                  30

Lys Arg Lys Leu Glu Arg Val Gln Ser Gly Leu Asp Leu Phe Thr Val
        35                  40                  45

Leu Leu Glu Gln Asn Asp Leu Glu Arg Gly His Thr Gly Leu Leu Arg
    50                  55                  60

Glu Leu Leu Ala Ser Leu Arg Arg His Asp Leu Leu Gln Arg Leu Asp
65                  70                  75                  80

Asp Phe Glu Ala Gly Thr Ala Thr Ala Ala Pro Pro Gly Glu Ala Asp
                85                  90                  95

Leu Gln Val Ala Phe Asp Ile Val Cys Asp Asn Val Gly Arg Asp Trp
            100                 105                 110

Lys Arg Leu Ala Arg Glu Leu Lys Val Ser Glu Ala Lys Met Asp Gly
        115                 120                 125

Ile Glu Glu Lys Tyr Pro Arg Ser Leu Ser Glu Arg Val Arg Glu Ser
    130                 135                 140

Leu Lys Val Trp Lys Asn Ala Glu Lys Lys Asn Ala Ser Val Ala Gly
145                 150                 155                 160

Leu Val Lys Ala Leu Arg Thr Cys Arg Leu Asn Leu Val Ala Asp Leu
                165                 170                 175

Val Glu Glu Ala Gln Glu Ser Val Ser Lys Ser Glu Asn Met Ser Pro
            180                 185                 190

Val Leu Arg Asp Ser Thr Val Ser Ser Ser Glu Thr Pro
        195                 200                 205

<210>  25

<211>  360

<212>  PRT

<213>  Homo sapiens

<400>  25

Met Ser Gln Glu Arg Pro Thr Phe Tyr Arg Gln Glu Leu Asn Lys Thr
1               5                   10                  15

Ile Trp Glu Val Pro Glu Arg Tyr Gln Asn Leu Ser Pro Val Gly Ser
            20                  25                  30

Gly Ala Tyr Gly Ser Val Cys Ala Ala Phe Asp Thr Lys Thr Gly Leu
        35                  40                  45

Arg Val Ala Val Lys Lys Leu Ser Arg Pro Phe Gln Ser Ile Ile His
    50                  55                  60

Ala Lys Arg Thr Tyr Arg Glu Leu Arg Leu Leu Lys His Met Lys His
65                  70                  75                  80

Glu Asn Val Ile Gly Leu Leu Asp Val Phe Thr Pro Ala Arg Ser Leu
                85                  90                  95

Glu Glu Phe Asn Asp Val Tyr Leu Val Thr His Leu Met Gly Ala Asp
            100                 105                 110

Leu Asn Asn Ile Val Lys Cys Gln Lys Leu Thr Asp Asp His Val Gln
        115                 120                 125

Phe Leu Ile Tyr Gln Ile Leu Arg Gly Leu Lys Tyr Ile His Ser Ala
    130                 135                 140

Asp Ile Ile His Arg Asp Leu Lys Pro Ser Asn Leu Ala Val Asn Glu
145                 150                 155                 160

Asp Cys Glu Leu Lys Ile Leu Asp Phe Gly Leu Ala Arg His Thr Asp
                165                 170                 175

Asp Glu Met Thr Gly Tyr Val Ala Thr Arg Trp Tyr Arg Ala Pro Glu
            180                 185                 190

Ile Met Leu Asn Trp Met His Tyr Asn Gln Thr Val Asp Ile Trp Ser
        195                 200                 205

Val Gly Cys Ile Met Ala Glu Leu Leu Thr Gly Arg Thr Leu Phe Pro
    210                 215                 220

Gly Thr Asp His Ile Asp Gln Leu Lys Leu Ile Leu Arg Leu Val Gly
225                 230                 235                 240

Thr Pro Gly Ala Glu Leu Leu Lys Lys Ile Ser Ser Glu Ser Ala Arg
                245                 250                 255

Asn Tyr Ile Gln Ser Leu Thr Gln Met Pro Lys Met Asn Phe Ala Asn
            260                 265                 270

Val Phe Ile Gly Ala Asn Pro Leu Ala Val Asp Leu Leu Glu Lys Met
        275                 280                 285

Leu Val Leu Asp Ser Asp Lys Arg Ile Thr Ala Ala Gln Ala Leu Ala
    290                 295                 300

His Ala Tyr Phe Ala Gln Tyr His Asp Pro Asp Asp Glu Pro Val Ala
305                 310                 315                 320

Asp Pro Tyr Asp Gln Ser Phe Glu Ser Arg Asp Leu Leu Ile Asp Glu
                325                 330                 335

Trp Lys Ser Leu Thr Tyr Asp Glu Val Ile Ser Phe Val Pro Pro Pro
            340                 345                 350

Leu Asp Gln Glu Glu Met Glu Ser
        355                 360

<210>  26

<211>  360

<212>  PRT

<213>  Mus musculus


<400>  26

Met Ser Gln Glu Arg Pro Thr Phe Tyr Arg Gln Glu Leu Asn Lys Thr
1               5                   10                  15

Ile Trp Glu Val Pro Glu Arg Tyr Gln Asn Leu Ser Pro Val Gly Ser
            20                  25                  30

Gly Ala Tyr Gly Ser Val Cys Ala Ala Phe Asp Thr Lys Thr Gly His
        35                  40                  45

Arg Val Ala Val Lys Lys Leu Ser Arg Pro Phe Gln Ser Ile Ile His
    50                  55                  60

Ala Lys Arg Thr Tyr Arg Glu Leu Arg Leu Leu Lys His Met Lys His
65                  70                  75                  80

Glu Asn Val Ile Gly Leu Leu Asp Val Phe Thr Pro Ala Arg Ser Leu
                85                  90                  95

Glu Glu Phe Asn Asp Val Tyr Leu Val Thr His Leu Met Gly Ala Asp
            100                 105                 110

Leu Asn Asn Ile Val Lys Cys Gln Lys Leu Thr Asp Asp His Val Gln
        115                 120                 125

Phe Leu Ile Tyr Gln Ile Leu Arg Gly Leu Lys Tyr Ile His Ser Ala
    130                 135                 140

Asp Ile Ile His Arg Asp Leu Lys Pro Ser Asn Leu Ala Val Asn Glu
145                 150                 155                 160

Asp Cys Glu Leu Lys Ile Leu Asp Phe Gly Leu Ala Arg His Thr Asp
                165                 170                 175

Asp Glu Met Thr Gly Tyr Val Ala Thr Arg Trp Tyr Arg Ala Pro Glu
            180                 185                 190

Ile Met Leu Asn Trp Met His Tyr Asn Gln Thr Val Asp Ile Trp Ser
        195                 200                 205

Val Gly Cys Ile Met Ala Glu Leu Leu Thr Gly Arg Thr Leu Phe Pro
    210                 215                 220

Gly Thr Asp His Ile Asp Gln Leu Lys Leu Ile Leu Arg Leu Val Gly
225                 230                 235                 240

Thr Pro Gly Ala Glu Leu Leu Lys Lys Ile Ser Ser Glu Ser Ala Arg
                245                 250                 255

Asn Tyr Ile Gln Ser Leu Ala Gln Met Pro Lys Met Asn Phe Ala Asn
            260                 265                 270

Val Phe Ile Gly Ala Asn Pro Leu Ala Val Asp Leu Leu Glu Lys Met
        275                 280                 285

Leu Val Leu Asp Ser Asp Lys Arg Ile Thr Ala Ala Gln Ala Leu Ala
    290                 295                 300

His Ala Tyr Phe Ala Gln Tyr His Asp Pro Asp Asp Glu Pro Val Ala
305                 310                 315                 320

Asp Pro Tyr Asp Gln Ser Phe Glu Ser Arg Asp Leu Leu Ile Asp Glu
                325                 330                 335

Trp Lys Ser Leu Thr Tyr Asp Glu Val Ile Ser Phe Val Pro Pro Pro
            340                 345                 350

Leu Asp Gln Glu Glu Met Glu Ser
        355                 360

## Description

Apoptosis, or programmed cell death, is a process involved in development and homeostasis. Fas and Fas Ligand (FasL), members of the tumour necrosis factor (TNF) receptors/TNF families, are implicated in this process. Although Fas is constitutively expressed on most cells of the body, FasL has a more restrictive pattern of expression. FasL is expressed on activated T lymphocytes, where it contributes to cytotoxicity and to the regulation of the immune response by activation-induced cell death (AICD). FasL is also expressed in Sertoli cells of the testis, and in cells of the anterior chamber of the eye, where it contributes to the immune privilege status of these sites by killing infiltrating lymphocytes that express the receptor Fas. Recently, a new concept has emerged showing that Fas/FasL interactions may not only induce apoptosis of invading cytotoxic lymphocytes, but may also stimulate their own proliferation through Fas/FasL interactions.

Dysregulation of the Fas pathway is responsible of hypo- or hyperproliferative disorders that are due to excess or default of apoptosis, respectively. Some reports suggest that FasL expression on tumour cells enables these cells to escape the immune system (counter-attack model), although this point is still controversial. Usually, when the tumour cells co-express Fas and FasL molecules, they turn out to be resistant to Fas-mediated cell death. Among the regulatory mechanisms that may take place to block the death signal, down-regulation of pro-apoptotic molecules like Fas or caspase 8, or up-regulation of anti-apoptotic molecule like FLIP (FLICE inhibitory protein) have been reported during tumour progression.

In susceptible cells, activation of Fas by its ligand leads to the subsequent recruitment of the adapter molecule FADD (Fas-associated protein with death domain) which in turn activates FLICE/caspase 8, a process that leads to apoptosis. FLIP, an anti-apoptotic protein, can protect cells from programmed cell death by preventing association of caspase 8 with the adapter molecule FADD.

Daxx (Fas death domain-associated protein), a second adapter molecule for the Fas receptor, defines a different signalling pathway downstream of Fas. It can activate independently the Jun N-terminal kinases (JNKs) or p38 mitogen-activated protein kinases (p38 MAPKs) pathways through the ASK1 (apoptosis signal-regulating kinase 1) intermediate. Whether Daxx signalling leads to induction of death or proliferation is not clear. It seems that JNK pathway generally leads to apoptosis whereas p38 kinase have been described as having pro- or anti-apoptotic effects. The final result therefore appears to depend from multiple factors such as the type of cells and its state of differentiation.

The kinase RIP (receptor-interacting protein), was recently shown to be a third adapter for Fas that can transduce Fas-mediated caspase-dependent or -independent cell death, further increasing the complexity of the Fas signalling pathway.

Faced with the still increasing complexity of the Fas/FasL pathway transducing machinery, the inventors first made the choice of a particular model in which Fas/FasL interactions have a particularly intriguing role, namely the thyroid organ. Indeed, in thyroid follicular cells (TFC), the role of the Fas pathway is highly debated, and has been the focus of multiple studies. The overall idea is that normal thyrocytes constitutively express the Fas molecule, and that FasL expression can occur under numerous thyroid pathological conditions including Hashimoto's thyroiditis (HT), Grave's disease and cancer. Whether thyrocyte FasL expression is beneficial or detrimental is more confusing and certainly depends from the type of thyroid disease. For example, in Hashimoto's thyroiditis (HT), Fas-FasL interactions among HT thyrocytes could lead to suicide/fratricide of the cells, and consequent hypothyroidism. By contrast, FasL expression by TFC in cancer and Grave's disease has been postulated to help the thyroid cells to escape immune system. One observation which has held the inventors' attention is that TFC are resistant to Fas-mediated cell death under normal conditions, and that some reports indicated that massive Fas-mediated apoptosis of TFC could be obtained after treatment with IL-1beta or IFNgamma, whereas other reports indicated that they were unable to induce cell death under the same conditions, but could succeed with a combination of IL-1beta and IFNgamma, while some still other reports indicated that IL-lbeta and/or IFNgamma do not induce TFC apoptosis, but rather a blockage of proliferation. Hence, due to the controversy arising from non confluent experimental results, it has been difficult to address experimentally the issue of Fas pathway regulation in general, and of Fas pathway regulation in the thyroid organ in particular.

Faced with these technical problems, the inventors then made the further choice to direct their focus towards *in vivo* models, namely non-human mammals which have been genetically engineered so as to develop thyroid tumours. They notably made the choice of using transgenic mice which develop tumour through a constitutive activation of adenylate cyclase. These mice have no abnormality up to 8 months of age, and thereafter develop hyperplasia followed by hyper-functioning thyroid adenomas, or adenocarcinomas depending of the genetic background of the mice. Such a constitutive activation of adenylate cyclase is observed in approximately 30% of human thyroid toxic adenomas and 10% of human thyroid carcinomas. The inventors thereby selected a non-human model which has the particular feature of being a powerful model for studying human tumours.

The inventors then further chose to investigate a large array of those signalling molecules involved in the Fas cascade in gsp transgenic mice, and to combine three different techniques (semi-quantitative RT-PCR, western blot and immunohistochemistry) to detect and quantify molecules at both mRNA and protein contents, and to localize protein expressing cells.

The results thus obtained show that, under *in vivo* conditions, normal thyroid follicular cells (TFC) of non transgenic (NTG) mice, like non pathological gland of transgenic mice, do not express FasL molecule. By contrast, FasL expression is acquired during *in vivo* tumoural development with a higher expression in adenoma/adenocarcinoma than in hyperplastic thyroids. This up-regulation of FasL on tumour cells is correlated with a down-regulation of molecules of the Fas signalling pathway.

Among the three known Fas receptor adapters (FADD, Daxx, RIP), only FADD was highly regulated. Moreover, this FADD regulation during tumour development has a very striking feature: indeed, the inventors found that although FADD mRNA was present, FADD protein expression was very weak or completely lost in tumour.

The inventors have furthermore observed that if *ex vivo* normal thyroid cells express both FADD mRNA and protein, they however loose FADD expression when placed under standard *in vitro* TFC culture conditions. They have also observed that some tumour cell lines, such as for example the K562 cell line, express FADD, whereas the corresponding *ex vivo* tumour, such as CML (chronic myeloid leukaemia) in the case the K652 cell line, does in fact not express FADD. Those prior art results which were based on *in vitro* cultures, or on established cell lines cannot therefore be directly transposed to the actual *in vivo* conditions. Apart from evidencing that such prior art experiments have suffered from important artefacts, the inventors thereby demonstrate that, in the absence of FADD, Fas-signalling results in faster cell growth (and not in apoptosis).

The inventors also demonstrate that during *in vivo* tumour development, Fas signalling, which is deprived of FADD, acts mainly through a Daxx-ASK1 pathway, ASK1 expression being strongly increased during tumour growth. As to the downstream kinases of this Daxx-ASK1 pathway, the inventors demonstrate that the ASK1/SEK1/JNK pathway, as well as the ASK1/MKK3/p38-MAPK pathway are strongly inhibited during *in vivo* tumour development. More particularly, they show that during *in vivo* tumour development:
- there is a decrease of total MKK3 expression,
- among MKK3/MKK6 proteins, only MKK6 proteins are activated,
- the activation of p38-MAPK is inhibited (decrease of phospho-p38-MAPK), and that this inhibition correlates with the decrease in total MKK3 expression,
- the expression content of total SEK1 proteins strongly increases, but the content of the thr261 phospho-active form of SEK1 strongly decreases, and that
- SAPK/JNK proteins are phosphorylated at both thr183 and thr185 sites in non pathological organs, but that hyperplastic organs and tumours are mono-phosphorylated due to a lack of phospho-SEK1 dependent thr183 phosphorylation.

The inventors have thus demonstrated that during the transformation process from non pathological organ to tumour:
1) FasL expression is gained,
2) FADD protein but not FADD mRNA is lost,
3) Daxx and RIP contents are not significantly affected,
4) Fas signalling in the absence of FADD results in a Daxx- ― but not RIP- ― mediated signal,
5) there is an increase in ASK1 expression content (Daxx-ASK1 pathway),
6) disappearance of FADD protein is correlated with an inactivated status of p38-MAPK (inhibition of p38-MAPK phosphorylation),
7) there is a switch from dual to mono-phosphorylation of SAPK/JNK proteins
8) there is an increase in total SEK1 protein expression content, and with a decrease in the content of thr261 phospho-active form of SEK1,
9) Fas signalling in absence of FADD promotes cell growth instead of apoptosis.

This is the first instance where spontaneous FADD protein disappearance has been associated with actual *in vivo* pathological consequences, and that a decrease in phosphorylated p38-MAPK proteins has been determined as closely correlated with this decrease in FADD proteins.

Indeed, prior art results on FADD solely concerned models which had been engineered by man to have a deficient FADD function. Models based on gene targeting, or on expression of dominant negative interfering mutant have thus been described on T lymphocytes, and were shown to render T cells resistant to apoptosis induced by Fas (Newton *et al.* 1998, The EMBO Journal 17(3):706-718). In these models, engineered FADD defects also resulted in an inhibition of mitogen-induced T-cell proliferation, demonstrating that FADD may transmit both apoptotic and growth and/or survival signals. Conversely, another model demonstrated that an engineered FADD deficiency in rag-1^{-/-} mice induced the development of thymic lymphoma, indicating that FADD may also act as a lymphoma suppressor (Newton *et al.,* 2000, The EMBO Journal Vol. 19 No.5 pp. 931-941). These prior art models mainly demonstrated that FADD protein might have various effects depending on the time of its expression, the tissue and the development status of the tissue.

In the present *in vivo* tumour model, the inventors demonstrate that there is a spontaneous loss of FADD expression, and more particularly that FADD undergoes post-translational regulations during the development of the tumour. This is the first description that the decrease in FADD proteins is a reliable marker of tumour progression, and there is a closely correlated decrease in phosphorylated p38-MAPK.

None of the prior art models, in which FADD function was deliberately altered by man, so as to elucidate the mechanisms by which Fas is regulated, does suggest that there is an actual loss in FADD expression during *in vivo* tumoural progression, that this loss affects FADD protein but not FADD mRNA, and that there may be a correlated decrease in phosphorylated p38-MAPK. Of particular note in this respect is that, according to the present invention, there is no significant regulation of Daxx and RIP, whereas Daxx and RIP have been described in prior art models as apoptosis potentiators (see *e.g.* US 5,674,734 Leder *et al.* ; US 6,159,731 Yang *et al*.). This illustrates that one cannot deduce from models which have been engineered to be deficient in a particular molecule, and which thereby show that this particular molecule favours (or, on the contrary, prevents from) a tumour development, that this particular molecule actually is a biological marker of tumour development (or regression, respectively).

The results obtained by the inventors on a non-human mammal model of adenoma/adenocarcinoma have further been confirmed on human patients, and on other types of tumours, such as notably acute and chronic myeloid leukaemia (see example 2 below): the decrease in FADD protein expression measured in a blood sample collected from a myeloid leukaemia patient reliably correlates with the aggressiveness of the disease. These results hence altogether fully confirm that the decrease in FADD proteins and/or in phosphorylated p38-MAPK is a reliable marker for the *in vivo* development of tumours such as adenoma, adenocarcinoma, myeloid leukaemia, and that it is applicable to human persons as well as to non-human animals.

The present patent application thus generally relates to the use of a protein selected from the group consisting of FADD proteins and phosphorylated p38-MAPK, as a marker of which absence or abnormally low content indicates that a tumour is present, or in development, or in spreading in an animal. In addition to the detection of a decrease in FADD proteins and/or phosphorylated p38-MAPK, it is according to the invention advantageously further made use of FasL protein as a marker of which gained expression indicates that a tumour is present, or in development, or in spreading in an animal. It has however to be noted that the adapter FADD is not exclusively involved in the Fas pathway: FADD is also involved in the pathways of a wide variety of other death receptors, such as TNFR1, DR3, DR4 and DR5.

The invention more particularly relates to methods and kits which are helpful for diagnosing the presence of a tumour in an animal, for determining the *in vivo* efficiency of a tentative anti-tumour treatment, and for determining the danger rating a compound may have *in vivo* in terms of pro-tumour activity. All these methods and kits have in common that the disappearance of FADD proteins and/or the correlated disappearance of phosphorylated p38-MAPK have been identified by the inventors as reliable markers of the *in vivo* presence or development of a tumour.

As above-indicated, and as further illustrated in the below examples, the level of FADD expression as well as of the content in phosphorylated p38-MAPK are indeed most generally high in non-tumour animal tissue or fluid, and drastically decrease, sometimes down to zero, when a tumour develops from these tissue or fluid. As a parallel feature, expression of FasL is gained.

The present method therefore encompasses a method and a kit to determine whether cells have or not turned into tumour cells in an animal, characterized in that:
at least one content selected from the group consisting of the content in FADD proteins and
the content in phosphorylated p38-MAPK is measured in said cells, and
wherein said cells are determined as tumour cells, when said measured content(s) either is(are) not significantly different from zero, and/or is(are) significantly inferior to the standard normal (respective) content(s) which is(are) observed in non-tumour but otherwise equivalent cells, and
wherein said cells are determined as non-tumour cells, when said measured content(s) is(are) significantly different from zero, and either not significantly different from, or significantly superior to said standard (respective) normal content(s).

By content in FADD and content in phosphorylated p38-MAPK, it is herein meant concentration in FADD proteins and in phosphorylated p38-MAPK proteins, respectively, as well as any other measurable value that may reflect the level of FADD expression, or the level of p38-MAPK proteins which are phosphorylated, respectively.

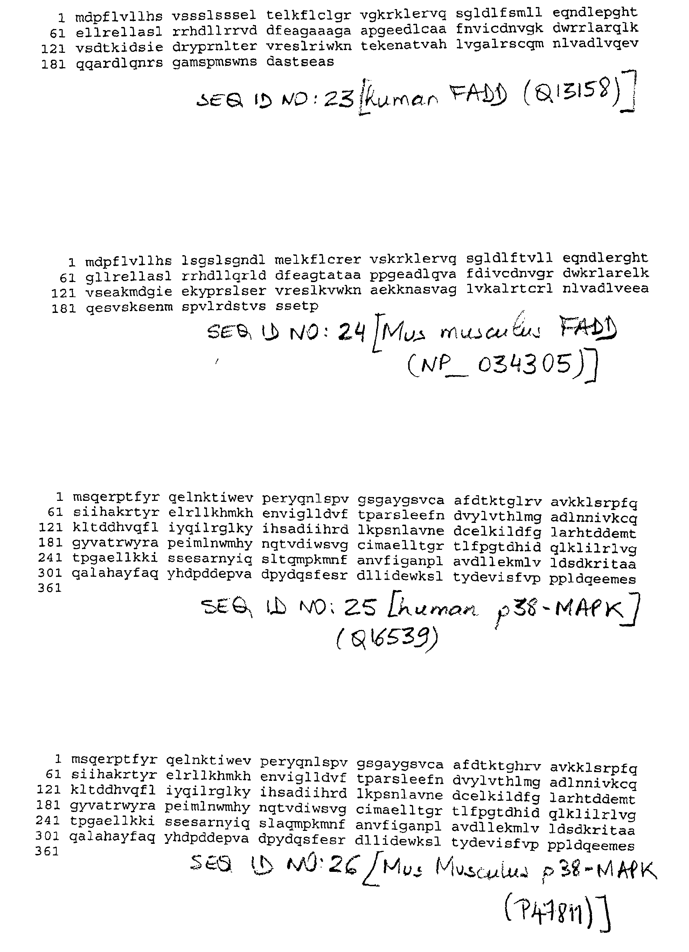

FADD proteins have an apparent MW of 28-30 kDa on Western blot, and have the amino acid sequence SEQ ID NO:23 in humans (accession Q13158), SEQ ID NO:24 in mice (accession NP_034305).
Phosphorylated p38-MAPK have an apparent MW of 42-44 kDa on western blot, and p38-MAPK has the amino acid sequence SEQ ID NO:25 in humans (accession Q16539), SEQ ID NO:26 in mice (accession P47811). SEQ ID NO:23-26 are also shown on Figure 7.

FADD as well as phosphorylated p38-MAPK have an ubiquitous panel of expression under physiological conditions, which implies that the vast majority of cells collected from an animal tissue or fluid should express FADD proteins and contain phosphorylated p38-MAPK when they have a non-tumour status, and should have a decreased content in, or an absence of FADD proteins and of phosphorylated p38-MAPK, when they develop a tumour status. The method of the invention is therefore pertinent to the vast majority of animal cells, except those cells which would, by exception, not express FADD under tumour-free normal conditions, and would not have phosphorylated p38-MAPK under activated but tumour-free normal conditions. It is at this stage reminded that, as above explained and below illustrated, it is of first importance for the present invention, to consider those FADD and/or phospho-p38-MAPK contents which are actually present *in vivo,* for example by analysing an *ex vivo* sample, and to not directly rely on measures performed on *in vitro* cultures or on established cell lines as these later experimental conditions may introduce artefacts in this respect (this is notably the case for *in vitro* standard cultures of non-tumour thyrocytes).

The skilled person is therefore invited to proceed to the usual control analysis which is the standard procedure in biotechnology, so as to check that when they do not have a tumour status, the tested tissue, fluid or cells actually express FADD proteins and/or phosphorylated p38-MAPK *in vivo,* thereby measuring those contents which are hereinafter referred to as the standard (respective) normal contents. Under non-tumour conditions, FADD should indeed be expressed, and p38-MAPK should be phosphorylated when the cell is activated.
The present application hence generally relates to the use of at least one protein selected from the group consisting of FADD proteins and phosphorylated p38-MAPK, as a marker for absence of *in vivo* tumour. This use may further be complemented by the use of the FasL protein as a marker for presence of *in vivo* tumour. The (respective) content(s) in FADD proteins and phosphorylated p38-MAPK indeed decrease sometimes up to zero with tumour development, while FasL expression is gained. All the methods and uses of the invention may therefore advantageously comprise the detection of the presence or absence, or the measure of the content in FasL proteins. Such a detection or measure can be achieved by anti-FasL specific compounds, such as anti-FasL specific antibodies (*e.g.* the anti-FasL IgG2A H11 manufactured by Alexis Biochemicals ; the rabbit polyclonal anti-FasL antibodies Q20 and C20 available from Santa Cruz Biotechnology ref.sc-956 and ref.sc-957, respectively ; the rabbit polyclonal anti-FasL (Ab-3) available from Oncogene Research ref. PC105 ; the mouse monoclonal anti-FasL (G247-4) available from BD PharMingen ref.65431A; the mouse monoclonal anti-FasL (NOK-1)BD PharMingen ref. 65321A ; the mouse monoclonal anti-FasL (NOK-2) available from BD PharMingen ref. 65331A).
Such *in vivo* tumours notably include benign tumours such as adenomas, as well as cancers such as adenocarcinoma, myeloid leukaemia. The absence of expression of, or an abnormally low content in FADD proteins and/or phosphorylated p38-MAPK is a particularly reliable marker for thyroid toxic adenomas, thyroid carcinomas, acute and chronic myeloid leukaemia. These markers can be used for detecting a tumour in an animal such as a mammal, and notably in a human person.

According to a first aspect of the invention, the application relates to a method for determining whether a suspected tumour has developed or spread or is in development or spreading in an animal, or whether such a tumour is absent or has regressed near to remission or recovery. This method is characterised in that it comprises the following steps:
- a biological sample collected from said animal as representative of said suspected tumour is provided,
- at least one content selected from the group consisting of the content in FADD proteins, and the content in phosphorylated p38-MAPK, is measured in said sample, and
wherein said tumour is determined as having developed or spread or as being in development or in spreading, when said measured content(s) either is(are) not significantly different from zero, and/or is(are) significantly inferior to the standard normal (respective) content(s) which is(are) observed in a non-tumour but otherwise equivalent sample, and
wherein said tumour is determined as absent or as in regression near to remission or recovery, when said measured content(s) is(are) significantly different from zero and either not significantly different from or significantly superior to said standard (respective) normal content(s),
provided that said standard (respective) normal content(s) is(are) significantly different from zero.

To implement this method, it is highly recommended to measure the standard (respective) normal content(s) which should be found in the particular sample being tested if it would not contain a tumour, so as to check that this(these) standard (respective) normal content(s) actually is(are) significantly different from zero (thereby confirming that the FADD and phosphorylated p38-MAPK markers are applicable to the particular case under analysis).

Such standard (respective) normal content(s) are also helpful in that they represent precise reference values with which the measured ones can be accurately compared (thereby enabling an accurate diagnosis). One should however note that such an accuracy is not always necessary: for example, a total absence in FADD proteins and/or phosphorylated p38-MAPK is by itself sufficient to conclude that a tumour is present or in development, if it has been checked that these proteins are present under non-tumour normal *in vivo* conditions.

Said biological sample may be any tissue or fluid which is representative of the suspected *in vivo* tumour *(e.g.* blood or bone marrow for a suspicion of leukaemia, tissue samples for a suspicion of adenoma or adenocarcinoma, etc.).

More particularly, the application relates to a method for determining whether a tumour is in a phase of development or of spreading in an animal, whether it is in a phase of stabilization, or whether it is in a phase of regression, remission or recovery, by comparison with a previously-determined tumour status, which is characterised in that it comprises the following steps:
- a first biological sample which is representative of said tumour is provided,
- at least one content selected from the group consisting of the content in FADD proteins, and the content in phosphorylated p38-MAPK, is measured in said first sample, this(these) measured content(s) being referred to as the measured first content(s),
- a second representative biological sample collected at a time which is later than the collection time of said first sample is provided,
- the content(s) which has(have) been measured in said first sample are then measured in said second sample, this(these) measured content(s) being referred to as the measured second content(s), and
wherein said tumour is determined as being in a phase of development or of spreading, when said measured second content(s) is(are) significantly inferior to said measured (respective) first content(s),
wherein said tumour is determined as being in a phase of stabilization, when said measured second content(s) is(are) not significantly different from said measured (respective) first content(s),
wherein said tumour is determined as being in a phase of regression, remission or recovery, when said measured second content(s) is(are) significantly superior to said measured (respective) first content(s),
provided that the standard normal (respective) content(s) which is(are) observed in a non-tumour but otherwise equivalent sample is(are) significantly different from zero.
After collection of said first sample, but before collection of said second samples, said animal may be subjected to a tentative anti-tumour therapy, such as chemotherapy.

According to a second aspect of the invention, the application relates to a method for determining whether a treatment is, or will be, a highly efficient anti-tumour treatment. This method determines whether a treatment is, or will be, efficient to treat or to arrest the growth of a tumour which is present or in development or in spreading in an animal, and is characterised in that it comprises the following steps:
- a biological sample which has been collected from said animal or from a representative biological model thereof (wherein a comparable tumour is present or in development or in spreading), during the course or at the end of said treatment, as a representative sample of said tumour is provided,
- at least one content selected from the group consisting of the content in FADD proteins and the content in phosphorylated p38-MAPK is measured in said sample, and
wherein said treatment is determined as efficient for said animal, when said measured content(s) is(are) not significantly different from the standard (respective) normal content(s) in a non-tumour but otherwise equivalent biological sample,
provided that said standard (respective) normal content(s) is(are) significantly different from zero.

This method can be performed on the animal itself, but is of course preferably performed on a representative model in which a comparable tumour is present or in development or in spreading, when such a model is available. Such a representative model is notably useful when the animal for which the treatment efficiency has to be determined is a human patient. Such a model may then be a non-human mammal wherein a comparable tumour is present or developing *(e.g.* a mouse, a rabbit, a dog or a monkey which has been genetically engineered to express the same type of tumour as is expressed in said human patient, for example the TRAMP mouse model for prostate cancer described in Greenberg *et al.,* 1995, Proc. Natl. Acad. Sci. USA. 92: 3439-3443, of which content is herein incorporated by reference).

To conclude "during the course" of a treatment that this treatment is inefficient, the skilled person will of course choose to analyse the contents in said tumour markers after the treatment has being applied for a time that is sufficiently long for this particular treatment to have a reasonable expectation of showing a significant anti-tumour activity on the particular animal being tentatively treated. Alternatively, or complementarily, the skilled person may choose to repeat this marker content analysis several times during the course of the treatment, thereby determining the effects of the treatment as a function of time of application, so as to decide whether said treatment is capable of developing beneficial effects in the body of said animal, or whether it is very much unlikely to do so. Determining the minimum duration that is necessary to reach a reliable conclusion with a minimum of deleterious side effects is a matter of experimental or clinical optimisation which belongs to the common general expertise of the physician or clinician.

A sharper method for determining whether a treatment is, or will be, a highly efficient anti-tumour treatment is according to the invention a method for determining whether a treatment is, or will be, efficient against a tumour which is present but not developing or spreading in animal, or whether it is or will be inefficient. It is characterised in that it comprises the following steps:
- a biological sample which has been collected from said animal or from a representative biological model thereof (wherein a comparable tumour is present) as representative of said tumour, during the course or at the end of said treatment is provided,
- at least one content selected from the group consisting of the content in FADD proteins and the content in phosphorylated p38-MAPK, is measured in said sample,
wherein said treatment is determined as efficient, when said measured content(s) is(are) significantly superior to the (respective) control content(s) which is(are) present in the absence of said treatment,
wherein said treatment is determined as inefficient, when said measured content(s) is(are) either significantly inferior to, or not significantly different from said (respective) control content(s),
provided that the standard normal (respective) content(s) which is(are) observed in a non-tumour but otherwise equivalent sample is(are) significantly different from zero.

More particularly, it is characterised in that it comprises the following steps:
- a first biological sample which is representative of said tumour is provided,
- at least one content selected from the group consisting of the content in FADD proteins and the content in phosphorylated p38-MAPK, is measured in said first sample, this(these) measured content(s) being referred to as the measured first content(s),
- said animal, or a representative biological model in which a comparable tumour is present, is subjected, after collection of said first sample, to said treatment to be tested,
- a second representative biological sample which has been collected from said animal or from said model as representative of said tumour during the course or at the end of said treatment is provided,
- the content(s) which has(have) been measured in said first sample are measured in said second sample, this(these) measured content(s) being referred to as the measured second content(s), and
wherein said treatment is determined as efficient, when said measured second content(s) is(are) significantly superior to said measured (respective) first content(s),
wherein said treatment is determined as inefficient, when said measured second content(s) is(are) either significantly inferior to, or not significantly different from said measured (respective) first content(s),
provided that the standard normal (respective) content(s) which is(are) observed in a non-tumour but otherwise equivalent sample is(are) significantly different from zero.

Another sharper method for determining whether a treatment is, or will be, a highly efficient anti-tumour treatment is according to the invention a method for determining whether a treatment is or will be efficient against a tumour which is in a phase of development or of spreading in an animal, or whether it is or will be inefficient. It is characterised in that it comprises the following steps:
- a biological sample which has been collected from said animal or from a representative biological model thereof (wherein a comparable tumour is in development or spreading) as representative of said tumour, during the course or at the end of said treatment, is provided,
- at least one content selected from the group consisting of the content in FADD proteins and the content in phosphorylated p38-MAPK, is measured in said sample,
wherein said treatment is determined as efficient, when said measured content(s) is(are) either significantly superior to the (respective) control content(s), which is(are) present in the absence of said treatment,
wherein said treatment is determined as inefficient, when said measured content(s) is(are) significantly inferior to said (respective) control content(s),
provided that the standard normal (respective) content(s) which is(are) observed in a non-tumour but otherwise equivalent sample is(are) significantly different from zero.

More particularly, it is characterised in that it comprises the following steps:
- a first biological sample which is representative of said tumour is provided,
- at least one content selected from the group consisting of the content in FADD proteins and the content in phosphorylated p38-MAPK, is measured in said first sample, this(these) measured content(s) being referred to as the measured first content(s),
- said animal, or a representative biological model in which a comparable tumour is in development or spreading, is subjected, after collection of said first sample, to said treatment to be tested,
- a second representative biological sample which has been collected from said animal or from said model as representative of said tumour during the course or at the end of said treatment is provided,
- the content(s) which has(have) been measured in said first sample are measured in said second sample, this(these) measured content(s) being referred to as the measured second content(s), and
wherein said treatment is determined as efficient, when said measured second content(s) is(are) either significantly superior to, or not significantly different from said measured (respective) first content(s),
wherein said treatment is determined as inefficient, when said measured second content(s) is(are) significantly inferior to said measured (respective) first content(s),
provided that the standard normal (respective) content(s) which is(are) observed in a non-tumour but otherwise equivalent sample is(are) significantly different from zero.

Given that when an anti-tumour treatment is efficient, the treated animal is said to be susceptible to this treatment, and that when an anti-tumour treatment is or becomes inefficient, the tentatively treated animal is said to be or to become resistant to this treatment, the method of the invention to determine whether a treatment is efficient or not can also be considered as a method to determine whether an animal, wherein a tumour is present, or in development or in spreading, is susceptible to a tentative anti-tumour treatment, or whether this animal is resistant to this treatment. Quite similarly, given that the degree of resistance, or of susceptibility of the animal which is subjected to anti-tumour treatment is said to reflect the degree of aggressiveness of the tumour disease, the method to determine according to the invention whether a treatment is efficient or not can also be considered as a method to determine whether the tumour development has a high degree of aggressiveness (resistance to treatment, inefficient treatment), or whether it has a low degree of aggressiveness (susceptibility to treatment, efficient treatment).

These methods of the invention therefore allow to adjust and optimise the treatment as necessary, and most importantly avoid treatments the continuation of which will anyhow not succeed in treating or arresting the tumour, and may even be deleterious to the patient.

To detect whether FADD proteins and/or phosphorylated p38-MAPK proteins are present or absent in a sample, as well as to possibly detect at which content they are respectively present, the skilled person can use any standard procedure for protein detection and quantification (see e.g. Current Protocols in Protein Science Edited by Coligan John E., & al. 2001. John Wiley & sons inc.; and Current Protocols in Immunology. Edited by Coligan John E., & al. 2001. John Wiley & Sons Inc., of which contents are herein incorportaed by reference). Such a detection is usually made by using a compound which is capable of specifically binding to FADD proteins and/or to phosphorylated p38-MAPK.

The invention therefore relates to the use of a compound which is capable of specifically binding to FADD proteins and/or to phosphorylated p38-MAPK, or of a biological unit which is capable of producing such a compound, for determining whether there is or not a tumour in an animal, and/or whether an anti-tumour treatment is, or will be, efficient or not in an animal. It more particularly encompasses a method and a kit for determining whether there is or not a tumour in an animal, and/or whether an anti-tumour treatment is or will be efficient or not in an animal.

The method to produce according to the invention a product useful for determining whether there is or not a tumour in an animal, and/or whether an anti-tumour treatment is, or will be, efficient or not in an animal is characterised in that it comprises the step of obtaining said product by production of a compound which specifically binds to FADD and/or to phosphorylated p38-MAPK, possibly followed by binding a detection label to this anti-FADD and/or anti-phosphorylated p38-MAPK specific compound.

The kit of the invention for determining whether there is or not a tumour in an animal, and/or whether an anti-tumour treatment is, or will be, efficient or not in an animal is characterised in that it comprises:
- at least one compound which is capable of specifically binding to FADD proteins and/or to phosphorylated p38-MAPK, or at least one biological unit which is capable of producing such a compound, this at least one compound or biological unit being possibly contained in a physiological buffer, and/or possibly bound to a solid support, and
- appropriate instructions for using FADD proteins and/or phosphorylated p38-MAPK as marker of the absence of an *in vivo* tumour.

By a compound which is capable of specifically binding to FADD and/or phosphorylated MAPK, it is herein meant that this compound can show with its target(s) a binding reaction which is of the antibody-antigen type, when this compound is placed under conditions appropriate for antibody-antigen type reactions, whereas when placed under the same conditions, this compound does not show a significant reaction of the antibody-antigen type with those other non-target molecular compounds of an animal cell, preferably of a mammal cell, most preferably of a human cell. Such a specific compound does advantageously not cross-react with other proteins of the MAPKinase family and with proteins having a death domain which is homologue (homology of 80% or greater, preferably of 70% or greater, most preferably of 60% or greater) to the death domain of the FADD protein.

Examples of conditions appropriate for antibody-antigen type reactions include standard assay conditions for protein detection and/or quantification, e.g. standard ELISA conditions, immuno(histo)chemistry conditions, standard Western/Dot/Slot blot conditions (see the below examples for illustrative immunochemistry and Western blot standard procedures, see also Harlow *et al.* ("*Antibodies - A Laboratory Manual*", Cold Spring Harbor Laboratory, 1988, edited by Edward Harlow and David Lane of which entire content is herein incorporated by reference ; Les Editions Inserm. 1987. Techniques en Immunologie. Techniques Immuno-enzymatiques. Th., Terninck & S., Avrameas. ; Current Protocols in Protein Science Edited by Coligan John E., & al. 2001. John Wiley & Sons Inc.; and Current Protocols in Immunology. Edited by Coligan John E., & al. 2001. John Wiley & Sons Inc. of which entire contents are herein incorporated by reference).

For said detection and/or quantification, said sample may be immobilized onto a solid support such as a membrane or a chip, or may be frozen and cut into sections.

Alternatively, the skilled person in the art may use FACS analysis to detect whether said binding compound finds a target in the analysed sample (see e.g. Current Protocols in Cytometry. Edited by J. Paul, Robinson. 2001. John Wiley & Sons Inc. of which entire content is herein incorporated by reference). For instance, permeabilization of the cells to be analysed could be performed and used for intracellular detection of target antigen.

Such specific compounds notably comprise antibodies specific for FADD proteins and/or against phosphorylated p38-MAPK. Said biotechnological unit can then be an hybridoma which is capable of producing such an antibody. This antibody may be monovalent or divalent, polyclonal or monoclonal. Examples of such antibodies include the polyclonal goat anti-FADD IgG commercialised by Santa Cruz Biotechnology with the product reference MC19, and the anti-phospho-p38-MAPK(thr180/tyr182) antibody commercialised by Cell Signalling Technology with the polyclonal goat anti-FADD IgG (MC-19) Santa Cruz Biotechnology ref. sc-6036; polyclonal goat anti-FADD IgG (N-18) Santa Cruz Biotechnology ref. sc-1172; polyclonal rabbit anti-FADD IgG (125-140) Calbiochem ref. 341282; polyclonal rabbit anti-phospho-p38-MAPK(thr180/tyr182) Cell Signalling Technology ref. 9211.

Other such antibodies can be produced by the person skilled person in the art according to standard methods in the field, described, for example, by Harlow *et al.* ("*Antibodies - A Laboratory Manual*", Cold Spring Harbor Laboratory, 1988, edited by Edward Harlow and David Lane), of which entire content is herein incorporated by reference.

Such an anti-FADD and/or anti-phospho-p38-MAPK specific antibody may also be a monoclonal antibody. For diagnosis applications, monoclonal antibodies are preferred. Appropriate monoclonal antibodies can be produced by conventional techniques, such as the one described by Köhler and Milstein, 1975 (Nature 256:495-497 *"Continuous cultures of fused cells secreting antibodies of predefined specificity*"), of which entire content is herein incorporated by reference. Examples of appropriate monoclonal antibodies include for example the anti-FADD mAbs commercialised by MBL International Corporation (200 Dexter Ave. Suite D Watertown, MA 02472, USA) [product references M033-3 (1F7, mouse IgG1) and MO35-3 (4G3, mouse IgG1)].

Such a compound may also be a fragment or a derivative of such an polyclonal or monoclonal antibody or diabody, provided that this fragment or derivative has retained an anti-FADD and/or auto-phospho-p38-MAPK specificity, as above defined. Examples of such fragments notably include F(ab')₂, Fab, Fv fragments. Examples of such derivatives notably include monovalent and divalent single chain antibodies (scFv), disulfide bond-stabilised Fv antibody fragments (dsFvs), bispecific dsFv-dsFv' constructs.

Such an anti-FADD and/or anti-phospho-p38-MAPK specific compound is thus obtainable, for example, by:
i.
   - immunization of an animal against a FADD protein and/or a phosphorylated p38-MAPK,
   - collection of the antibodies thus produced, and by
   - isolation of those antibodies which compete with anti-FADD and/or anti-phospho-p38-MAPK antibodies, such as anti-mouse FADD (MC-19) Santa Cruz Biotechnology ref. sc-6036 and/or anti-human FADD (125-140) Calbiochem ref. 341282 and/or anti-mouse and human phospho-p38-MAPK Cell Signalling Technology ref. 9211, for binding to FADD and/or phosphorylated p38-MAPK, and do not cross-react with other endogenous proteins, as above described.
   or by:
ii. - isolation of the Fv, Fab or F(ab')₂ fragments of such an anti-FADD and/or anti-phospho-p38-MAPK specific antibody(ies),
   or by:
iii. - grafting the CDRs fragments of such an anti-FADD and/or anti-phospho-p38-MAPK specific antibody(ies) onto a molecular structure which has the structure of an antibody backbone,
   or by:
iv. - operably cloning in a host cell, such as *E. coli,* the polynucleotide sequence of such an anti-FADD and/or anti-phospho-p38-MAPK specific antibody(ies), or of such a Fv, Fab or F(ab')₂ fragment, or of such a grafted molecular structure, and recovering the product produced by this host cell *via* the transcription and translation of said polynucleotide sequence.

Said kit of the invention for determining whether there is or not a tumour in an animal, and/or whether a tentative anti-tumour treatment is efficient or not may also comprise detection labels appropriate for detection of possible binding of said at least one binding compound to its target.

Examples of such detection labels include for example fluorescent labels or radioactive labels which may be coupled to the binding compound.

Other examples include secondary antibodies which can bind to said binding compound, such as horseradish peroxydase conjugated antibodies which enable to visualize such a binding after incubation on an ECL substrate (Amersham Pharmacia Biotech), or biotinylated conjugated secondary antibodies (Vector) which enable to visualize such a binding after incubation with phosphatase alcaline conjugated streptavidin (Amersham Life Science) on Fast-red substrate (Acros organics).

According to a further aspect of the invention, the application also relates to means for determining the danger rating a compound may have *in vivo* in terms of tumour and notably of cancer.

The application thus relates to a method for assessing whether a compound has a high or a low probability of having a tumour-inducing activity in a tumour-free animal. It is characterized in that it comprises the following steps:
- said compound is placed into contact with said tumour-free animal, or with a tumour-free representative biological model thereof,
- at least one content selected from the group consisting of the content in FADD proteins and the content in phosphorylated p38-MAPK, is measured in said animal or model, after said contact with said compound, and
wherein said compound is determined as having a high probability of having a tumour-inducing activity in said animal, when said measured content(s) is(are) significantly inferior to the standard (respective) normal content(s) which is(are) observed in the absence of contact with said compound,
wherein said compound is determined as having a low probability of having a tumour-inducing activity in said animal, when said measured content(s) is(are) not significantly inferior to said standard (respective) normal content(s),
provided that said standard (respective) normal content(s) is(are) significantly different from zero.

This method is useful for any animal and notably for mammals, and more particularly for human persons. For healthcare and safety purposes, a tumour-free representative biological thereof is preferably used for the implementation of the method. Such a representative biological model thereof can be an *in vivo* model such as *e.g* a mouse, a monkey, a rabbit, a dog, when said animal is a human person, or can be an *in vitro* model, such as a culture obtainable by culture of cells or tissue collected from said animal as representative of the susceptibility of said animal to develop a tumour, provided that the content in FADD proteins and/or in phosphorylated p38-MAPK does not decrease with time in this culture (which will be notably the case for *in vitro* standard culture of non-tumour thyrocytes).

Cultures should be placed and kept under conditions of temperature, medium composition, atmosphere (CO₂ content, O₂ content), humidity, light, which are appropriate for them to keep alive or to grow when they are not placed in contact with any candidate compound (control culture). Such cultures should not be thyrocyte cultures, as the inventors have observed that cultures of non-tumour thyrocytes under standard normal conditions induce a decrease in the content in FADD proteins and phosphorylated p38-MAPK ; it implies that the standard (respective) normal content(s) in markers of the invention gradually tends(tend), for such thyrocyte culture, toward a value which is(are) not significantly different from zero. Such cultures may however be, for example, hepatocyte cultures. The best way to proceed according to the invention is to check that the content in markers of the invention does not decrease with time for the particular culture which is chosen by the skilled person. Examples of candidate compounds to be tested notably include inhibitors of kinase functions.

The application also relates to a kit for assessing whether a compound has a high or a low probability of having a tumour-inducing activity in an animal. This kit is characterized in that it comprises at least one anti-FADD and/or anti-phospho-p38-MAPK specific compound, and possibly a tumour-free biological model, such as a cell culture or a cell culture lyophilisate.

As the inventors have demonstrated that several non-tumour primary cell cultures (such as non-tumour thyroid follicular cells (TFC)) or established cell lines loose FADD expression and/or phosphorylation of p38-MAPK under normal standard *in vitro* conditions, without being contacted with a tumour-inducing agent, the application also relates, according to a still further aspect of the invention, to the use of such an *in vitro* culture or established cell line, as an *in vitro* model to determine in the absence of any tumour-inducing agent whether a compound has an anti-tumour or anti-mitotic activity. The application more particularly relates to a method for determining whether a compound can have an anti-tumour or anti-mitotic activity in an animal. An example of such a method is characterised in that it comprises the following steps:
- non-tumour thyroid follicular cells (TFC) are placed for *in vitro* growth under conditions of substrate composition, of temperature and of time which enable the adhesion and confluence of said TFC,
- said compound is placed in contact with said culture,
- at least one content selected from the group consisting of the content in FADD proteins and the content in phosphorylated p38-MAPK is measured in said TFC after said contact with said compound, and
wherein said compound is determined as having an anti-tumour or anti-mitotic activity, when the measured content(s) is(are) either significantly superior to the (respective) content(s) which is(are) observed in a comparable TFC but in the absence of contact with said compound, or not significantly different from the (respective) content(s) which is(are) measured in *ex vivo* non-tumour TFC.

Said non-tumour TFC can be collected from any mammal including human persons, and are preferably collected from the same animal variety as the one for which the determination has to be made.

The contact duration which is necessary for said compound to show whether it has or not an effect of the content in FADD proteins and/or in phosphorylated p38-MAPK of course depends from the particular compound being tested. The minimum duration which is *a priori* necessary for obtaining significant results with a given compound usually belongs to the skilled person's average expertise. Alternatively, or complementary, the skilled person can choose to measure said at least one content as a function of time until said TFC stop growing.

Said non-tumour TFC intended for said *in vitro* culture may be submitted to a collagenase H treatment before being *in vitro* cultured.

Appropriate conditions for said in vitro growth of TFC notably, and strikingly, include normal standard culture conditions, such as 5-6 days at 37°C on RPMI 1640 medium supplemented with 5% fetal bovine serum.

Examples of candidate compounds notably include modulators of the MAP kinase pathways.

The application also relates to a kit for determining in the absence of any tumour-inducing agent whether a compound can have an anti-tumour or anti-mitotic activity in an animal. This kit is characterised in that it comprises:
- non-tumour thyroid follicular cells (TFC), or a lyophilisate thereof, and
- at least one anti-FADD and/or anti-phospho-p38-MAPK compound.

As non-tumour TFC grown *in vitro* loose FADD expression and p38-MAPK phosphorylation, the method, kit and use of the invention have the advantage of not requiring any tumour-inducing agent for their implementation.

In all the methods, kits and use of the invention, the content in FasL proteins (of which expression is gained with the development of a tumour) is advantageously measured in addition to detection of the content in FADD proteins and/or phosphorylated p38-MAPK, so as to determine whether this content increases compared to the standard normal FasL content which is observed in non-tumour but otherwise equivalent conditions, thereby confirming that a tumour is present or in development in the animal, and/or that the tentative anti-tumour treatment is inefficient, and/or that the tested compound has a high probability of having *in vivo* tumour-inducing activities, and/or that the compound has no anti-tumour or anti-mitotic *in vivo* activity.

The following examples are provided for the purposes of illustration, and are not intended to limit the scope of the present invention. They make various embodiments and advantages of the invention apparent to the skilled person. In these examples, reference is made to the following figures:

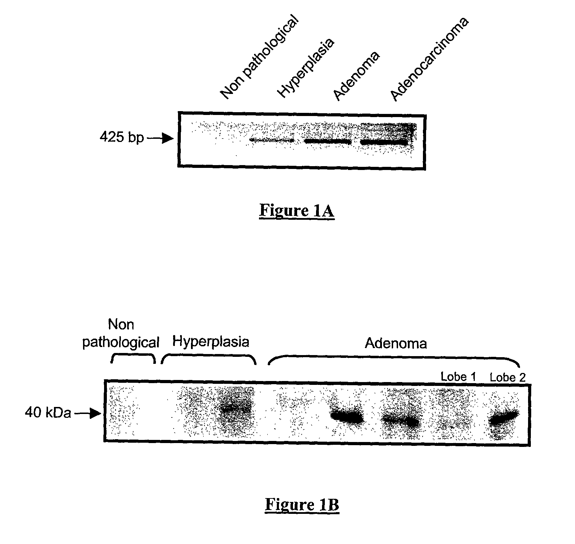

- Figures 1A and 1B illustrate that Fas ligand expression is acquired during tumour development. Figure 1A shows representative results of semi-quantitative RT-PCR of FasL mRNA from thyroid glands of gsp transgenic mice at various stages of tumour development, while Figure 1B shows the corresponding analysis for FasL proteins on Western blot. Amplified fragment and protein sizes are indicated. Differential FasL expression by the two lobes of the same adenoma is shown in lanes 7 and 8,

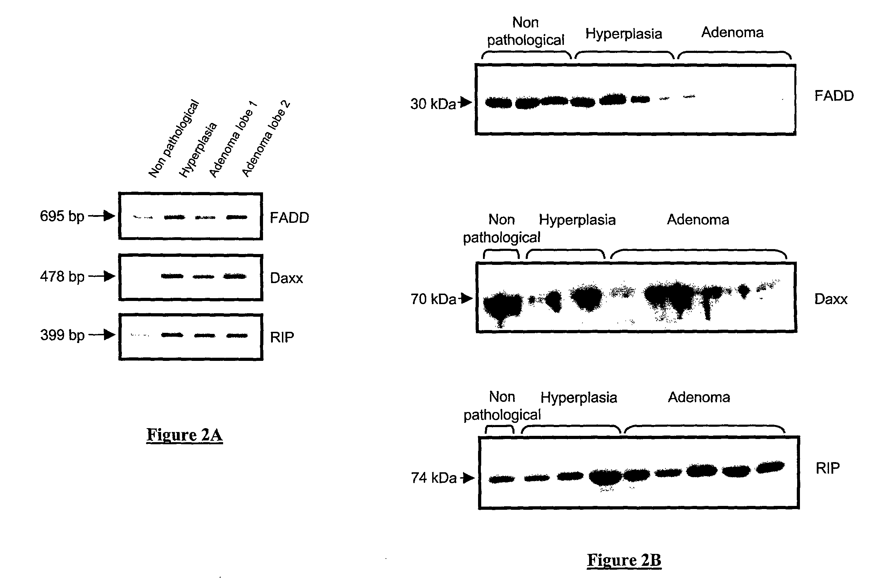

- Figures 2A and 2B illustrate that the adapter protein FADD is lost during tumour development. Figure 2A shows semi-quantitative RT-PCR analysis of FADD, Daxx and RIP mRNAs expression by thyroid glands from gsp transgenic mice at various stages of tumour development. The expression of the adapters mRNAs in the two lobes of the same adenoma is shown in lanes 3 and 4. Amplified fragments size is indicated. Figure 2B shows Western blot analysis of the three adapter molecules by thyroid glands from gsp transgenic mice at various stages of tumour development (FADD = panel 1 ; Daxx = panel 2 ; RIP = panel 3). Proteins size is indicated,

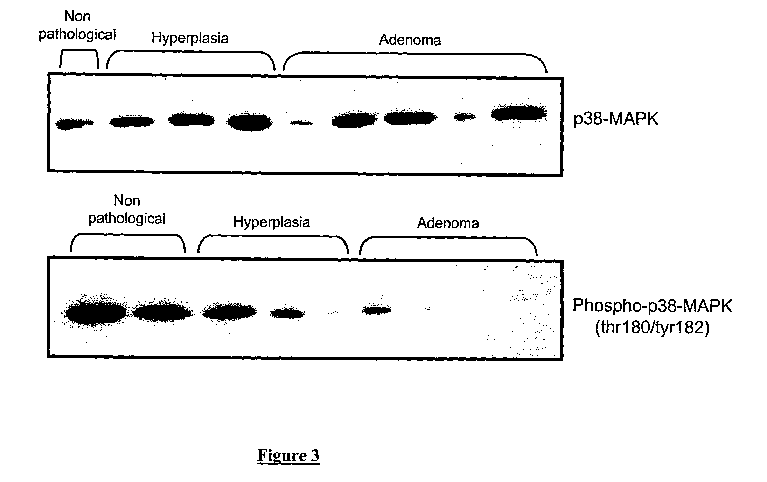

- Figure 3 illustrates that the p38-MAPK pathway is inhibited during tumour development, by showing Western blot analysis of total (upper panel) and active phosphorylated (lower panel) forms of p38-MAPK expression by thyroids glands from gsp transgenic mice at various stages of tumour development,

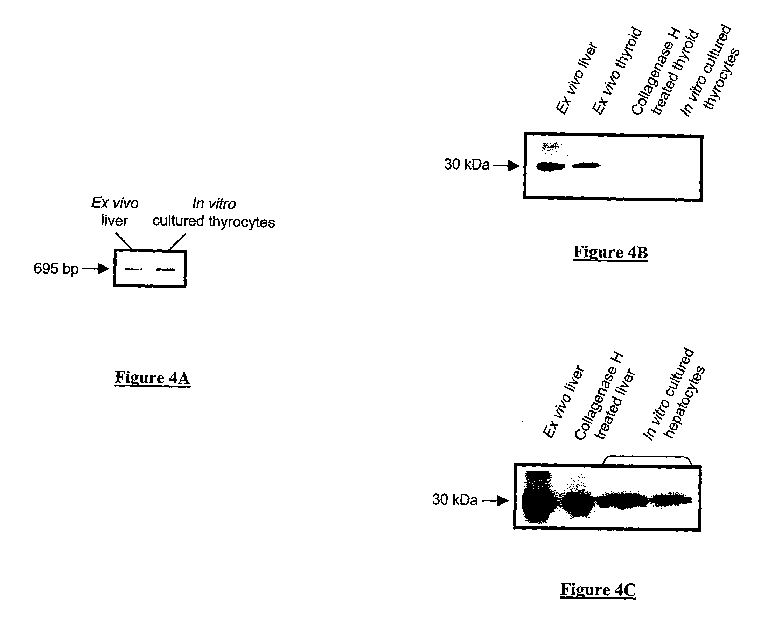

- Figures 4A, 4B and 4C illustrate that *in vitro* cultured thyrocytes do not express FADD protein. Figure 4A shows semi-quantitative RT-PCR analysis of FADD mRNA expression by *ex vivo* liver and *in vitro* culture of thyroid cells from normal non transgenic mice. Figure 4B and 4C show Western blot analysis of FADD protein expression by *ex vivo,* collagenase H treated and *in vitro* cultured thyrocytes (Figure 4B) or hepatocytes (Figure 4C) from normal non transgenic mice. Results of two independent *in vitro* cultures of hepatocytes are shown in lanes 3 and 4. Amplified fragment and protein sizes are indicated.

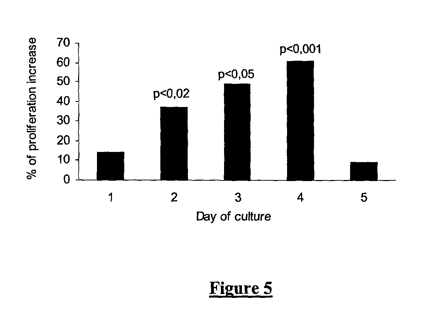

- Figure 5 illustrates that Fas signalling in absence of FADD increases TFC growth. Normal thyrocytes were cultured with or without clone Jo2 anti-Fas antibody for 24h to 120h exposure. Proliferation of thyrocytes was determined every day until confluence by adding [³H] thymidine for the last 12h of culture. Data represent percentage of proliferation increase of anti-Fas versus control cultures. Each point is mean cpm of six wells cultures. Value for proliferation of control TFC varied from 343 cpm to 1382 cpm. Value for proliferation of anti-Fas treated TFC varied from 392 cpm to 1507 cpm.

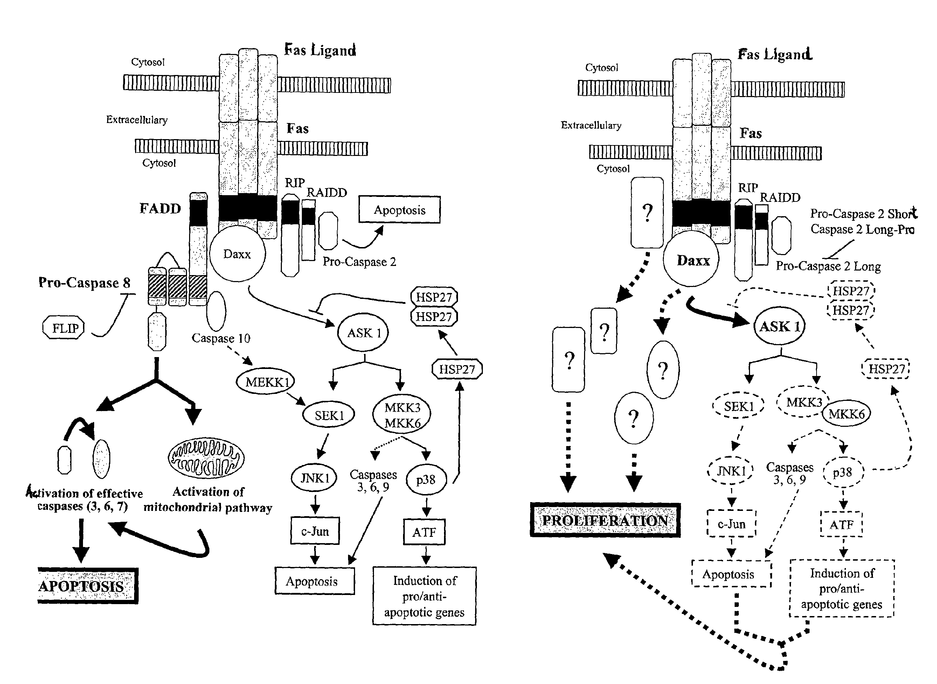

- Figure 6 is a schematic representation of the mechanisms by which the Fas pathway is regulated,
- Figure 7 shows human and *Mus musculus* FADD and p38-MAPK aminoacid sequences.

### EXAMPLE 1: when a tumour develops in a thyroid adenoma/adenocarcinoma non-human mammal model, FADD expression is lost (but not FADD mRNA), and there is a correlated decrease in phosphorylated p38-MAPK.

Due to the multiple possible artefacts and to the controversy existing on the detection of some of the molecules of the Fas cascade, we made the choice of investigating the presence of a large array of the signalling molecules of the Fas cascade in an *in vivo* tumour model, and to combine three different techniques (semi-quantitative RT-PCR, western blot and immunohistochemistry) to detect and quantify molecules both at the mRNA and protein levels and to localize protein expressing cells.
The *in vivo* tumour model we selected is a gsp transgenic mouse. In thyroid gland, guanine nucleotide stimulatory factor (Gs) activates adenylate cyclase in response to thyrotropin stimulation, leading to cyclic AMP level elevation and subsequent thyroid cells proliferation. Mutations of the α subunit of the Gs factor (gsp mutations), resulting in constitutive activation of adenylate cyclase, are found in approximately 30% of human thyroid toxic adenomas and 10% of human thyroid carcinomas. Transgenic mice expressing a mutant form of Gsα subunit directed to murine thyroid epithelial cells (gsp transgenic mice) are a powerful model for studying human thyroid tumours. These mice have no abnormality up to 8 months of age, and thereafter develop hyperplasia followed by hyperfunctioning thyroid adenomas (Michiels, 1994, PNAS Vol. 91 pp. 10488-10492), or adenocarcinomas depending from the genetic background of the mice.

Our results show that normal TFC of non transgenic (NTG) mice, like non pathological gland of transgenic mice, did not express FasL molecule. By contrast, FasL expression was acquired during tumour development with a higher expression in adenoma/adenocarcinoma than in hyperplastic thyroids. This up-regulation of FasL on tumour cells was correlated with a down-regulation of molecules of the Fas signalling pathway. Among the three adapters for Fas receptor, only FADD was highly regulated. Particularly, we found that although FADD mRNA was present, FADD protein expression was very weak or completely lost in adenoma/adenocarcinoma. Furthermore, by using an *in vitro* model of thyrocyte culture we found that, in the absence of FADD, Fas-signalling resulted in faster growth of thyrocytes apparently through a particular Daxx signalling.

### Materials and Methods

*Mice.* The gsp transgenic mice were described elsewhere (Michiels, 1994, reference cited supra). CBA/J female mice were purchased from Iffa Credo (L'Arbresle, France) and were used at 7-10 weeks of age in all experiments. All mice were maintained in standard environmental conditions with free access to food and water, and were allowed to adapt to their environment for one week before the experiments. Adenoma/adenocarcinoma development was monitored by measurement of T₄ and T₃ serum levels.

*Culture of primary thyrocytes.* Thyroid tissues were obtained from control CBA/J female mice. Animals were sacrificed and the two thyroid lobes cut off after intra-cardiac puncture to eliminate contaminating leukocytes. Collected lobes were digested in a 1.5mg/ml collagenase H (Boehringer Mannheim) solution for 30 minutes at 37°C. Thyroid cells were then cultured at 37°C in RPMI 1640 medium (Life Technologies) supplemented with 5% fetal bovine serum, 100U/ml penicillin (Life Technologies), 100µg/ml streptomycin (Life Technologies) and 5.10⁻⁵ 2-mercaptoethanol (complete culture medium). Non adherent cells were removed by washing to obtain TFC pure cultures. Cells reached sub-confluence after 5-6 days of culture.

*Culture of primary hepatocytes.* BALB/c hepatocytes were prepared according to Mazier *et al.* 1985 (Science Vol. 227 pp. 440-442) with minor modifications. Briefly, cells were isolated by perfusion of the liver fragments with collagenase (Boehringer Mannheim, Germany) following purification in a 60% Percoll gradient (Pharmacia Biotech, Uppsala, Sweden). Purified hepatocytes showed at least 95% of viability and purity assessed by Trypan blue dye exclusion. They were cultured in William's E medium (Gibco, Irvine, UK) supplemented with 5% of FCS (Gibco) and 1% of penicillin-streptomycin solution (100 X stock solution, Gibco) following incubation at 37°C for 24 h under 3.5% CO2 atmosphere.

*Semi-quantitative RT-PCR.* Thyroids of gsp transgenic mice were collected at various stages of tumour development and RNA was isolated from cells using 500µl to 1ml Tri-Reagent per thyroid (Molecular Research Center). For the *in vitro* culture model, TFC were cultured in 24-well flat bottomed plates (Costar) at approximately 10⁵ cells/well in a final volume of 1ml. At sub-confluence, thyroid cell cultures were washed with PBS and RNA was isolated from cells using 250µl/well Tri-Reagent. Contaminating DNA was eliminated by DNAse (Ozyme) treatment. Reverse transcription was performed on 2µg total RNA using Superscript II reverse transcriptase (Life Technologies). ADNc (50ng) was then amplified by polymerase chain reaction (PCR) using 0.5U Taq polymerase (Life Technologies). Specific primers used for PCR were: 
FasL (425-bp)	sense 5'-CAGCAGTGCCACTTCATCTTGG-3' (SEQ ID NO:1),
antisense 5'-TTCACTCCAGAGATCAGAGCGG-3' (SEQ ID NO:2),
 
Fas (366-bp) sense 5'-CTTCTACTGCGATTCTCCTG-3' (SEQ ID NO:3),
antisense 5'-CTTGCCCTCCTTGATGTTAT-3' (SEQ ID NO:4),
 
FADD (695-bp) sense 5'-TGGATGGGATTGAGGAGAAG-3' (SEQ ID NO:5),
antisense 5'-AAACCACAGTCCTCACAGGG-3' (SEQ ID NO:6),
 
Daxx (478-bp)          sense 5'-CCAACCTACCCCCCAATC-3' (SEQ ID NO:7),
                        antisense 5'-GCCTCAGAGGCAGTGTTTTC-3' (SEQ ID NO:8),
 
RIP (399-bp) sense 5'-CAGGAGCAAGAGGTCATTCTG-3' (SEQ ID NO:9),
antisense 5'-TGTTATCAAAGATGGCTTGGTG-3' (SEQ ID NO:10),
 
FLICE (488-bp)	sense 5'-GCTGACCTCAGATGTGAGAGG-3' (SEQ ID NO:11),
antisense 5'-TCCCATTGAAGATGTACACAGC-3' (SEQ ID NO:12),
 
FLIPL (389-bp)	sense 5'-TACAGAGTGAGGCGGTTTGA-3' (SEQ ID NO:13),
antisense 5'-AGCGAAGCCTGGAGAGTATT-3' (SEQ ID NO:14),
 
Bcl2 (290-bp) sense 5'-CGAGAAGAAGGGAGAATCAC-3' (SEQ ID NO:15),
antisense 5'-CATAAGGCAACCACACCATC-3' (SEQ ID NO:16),
 
Bcl-xl, Bad (361-bp)  sense 5'-AGCCCAGAGTATGTTCCAGA-3' (SEQ ID NO:17),
antisense 5'-CTCAAACTCATCGCTCATCC-3' (SEQ ID NO:18),
 
Bax (273-bp)	sense 5'-TGCTACAGGGTTTCATCCAG-3' (SEQ ID NO:19),
antisense 5'-CAAAGTAGAAGAGGGCAACC-3' (SEQ ID NO:20).
 βactin [sense 5'-TGGAATCCTGTGGCATCCATGAAAC-3' (SEQ ID NO:21), antisense 5'-TAAAACGCAGCTCAGTAACAGTCCG-3' (SEQ ID NO:22)] was used as control. PCR products were loaded onto a 2% agarose gel and visualized by staining with ethidium bromide. For quantification serial two fold dilution of cDNA were amplified for 25 cycles using βactin primers. cDNA giving equivalent amount of βactin were then amplified for 24 to 30 PCR cycles to ascertain that equal amount of cDNA were used.

*Western Blot.* Thyroids of gsp transgenic mice were collected at various stages of tumour development and total proteins were extracted with 200µl to 1ml lysis buffer (10mM Tris-HCl, 150mM NaCl pH7,8, 1%NP40, 1mM PMSF, 1µg/ml aprotinin and 1µg/ml leupeptin) containing protein phosphatase inhibitors (1nM cypermethrin, 30µM dephostatin, 50nM okadaic acid, 20nM tautomycin and 1mM orthovanadate) (Calbiochem). For the *in vitro* culture model, TFC were cultured in 6-well flat bottomed plates (Costar) at approximately 250,000 cells/well in a final volume of 2ml. At sub-confluence, thyroid cell cultures were washed with PBS and total proteins were extracted with 500µl/well of lysis buffer. Samples concentrations were determined using micro BCA protein assay reagent kit (Pierce). Electrophoresis through SDS-polyacrylamide gel was performed using 25µg of total proteins, and then transferred to nitrocellulose (NEN). The membranes were blocked with 5% milk in TBS 0.1% Tween 20 for one hour at room temperature (RT). Specific proteins were detected using specific primary antibodies at appropriate concentration: anti-mouse FasL (rat IgG2a; H11) was manufactured by Alexis Biochemicals; anti-mouse Fas (rabbit IgG; FL-335); FADD (goat IgG; MC19); Daxx (rabbit IgG; M112); RIP (rabbit IgG; H-207); caspase 10 (rabbit IgG; H-131); ASK1 (goat IgG; N-19); SEK1/MKK4 (rabbit IgG; C-20); PTEN (goat IgG; N-19); HSP27 (goat IgG; M-20); Apaf1 (goat IgG; N-19); and Bc12 (rabbit IgG; Δc21) were all obtained from Santa Cruz Biotechnology; anti-mouse caspase 8 and Bax (both rabbit IgG) were from Chemicon; anti-mouse FLIP (rabbit IgG) was from Euromedex and anti-mouse caspase 3 proform (goat IgG; 46) was from Transduction Laboratories; anti-mouse caspase 2 long and short (mouse IgG1; G310-1248) and Bcl-xl (rabbit IgG) were both from Pharmingen; anti-mouse phospho-SEK1/MKK4 (thr261); SAPK/JNK; phospho-SAPK/JNK (thr183/tyr185); MKK3; phospho-MKK3/MKK6 (ser189/207); p38-MAPK; phospho-p38-MAPK (thr180/tyr182); Akt; phospho-Akt (ser473); and Bad were all rabbit IgG obtained from Cell signalling Technology. After washes with TBS 0.1% Tween 20, membranes were incubated with horseradish peroxydase conjugated secondary antibodies for 1h at RT. After washing, the blots were developed using an ECL substrate (Amersham Pharmacia Biotech).
*Immunohistochemistry.* Immunohistochemical staining of 5µm frozen fixed tissue sections was performed using primary antibodies at 10µg/ml for 1h at RT. Biotinylated conjugated secondary antibody (Vector) was followed by phosphatase alcaline conjugated streptavidin (Amersham Life Science). Positive reactivity was revealed by adding Fast-red substrate (Acros organics). Staining positivity and intensity was assessed by two persons by blind evaluation of thyroid specimens.
*Proliferation assay.* TFC were cultured in 96-well flat bottomed plates (Costar) at approximately 125,000 cells/ml in a final volume of 150µl in absence or presence of clone Jo2 anti-Fas antibody (0.5µg/ml, Pharmingen). Proliferation of thyrocytes was determined from 24h to 120h. Thyrocytes were pulsed with 0.5µCi of [³H] thymidine during the last 12h of culture. Results are expressed as mean cpm of six wells cultures.

### Results

The results obtained are summarized in the below table 1:

**Table 1:**

| **mRNA and protein levels of the Fas cascade molecules from normal to pathological conditions** | | | | | | |
|---|---|---|---|---|---|---|
| | Non pathological thyroid | | Hyperplastic thyroid | | Adenoma Adenocarcinoma | |
| | mRNA | Protein | mRNA | Protein | mRNA | Protein |
| Fas Ligand | - | - | + | + | ++ or | ++ or +++ |
| Fas | + | + | + | + | + | + |
| FADD | + | +++ | + | +++or++ | + | +/- or - |
| DAXX | + | + | + | + | + | + |
| RIP | + | + | + | + | + | + |
| Caspase 8 | +/- | +/- | +/- | +/- | +/- | +/- |
| FLIP | + | ND | + | ND | + | ND |
| Caspase 10 | ND | + | ND | + | ND | + |
| Pro-Caspase 3 | ND | + | ND | + | ND | + |
| Pro-Caspase 2 Long | ND | ++ | ND | ++ or +++ | ND | ++ or +++ |
| Pro-Caspase 2 Short | ND | - | ND | - | ND | - or +/- |
| Caspase 2 Long Pro | ND | - | ND | - or + | ND - | or + or ++ |
| ASK1 | ND | + | ND | + or ++ | ND | + or ++ |
| SEK1/MKK4 | ND | + | ND | + | ND | + or ++ |
| Phospho-SEK1 | ND | + | ND | +/- | ND | +/- |
| SAPK/JNK | ND | + | ND | + | ND | + |
| Phospho-SAPK/JNK | ND | + (2P) | ND | + (1P) | ND | + (1P) |
| MKK3 | ND | +++ | ND | + | ND | + or +/- |
| Phospho-MKK3/6 | ND | +++ | ND | +++ | ND | +++ |
| P38-MAPK | ND | ++ | ND | +++ | ND | + or +++ |
| Phospho-P38-MAPK | ND | +++ | ND | + or +/- | ND | +/- or - |
| PTEN | ND | + | ND | + | ND | + |
| Akt | ND | + | ND | + | ND | + |
| Phospho-Akt | ND | + | ND | + | ND | + |
| HSP 27 | ND | + | ND | + | ND | + |
| Apaf 1 | ND | + | ND | + | ND | + |
| Bcl2 | + | + | + | + | + | + |
| Bcl-xl | + | + | + | + | + | ++ |
| Bad | + | + | + | + | + | + |
| Bax | + | + | + | + | + | ++ |
| - or +/-: null or very low band intensity, | | | | | | |
| +: low band intensity, | | | | | | |
| ++: medium band intensity, | | | | | | |
| +++: high band intensity. | | | | | | |

### Fas ligand expression is acquired during tumour development.

As FasL expression was reported on various cancer cells including human thyroid carcinoma, the presence of this molecule was investigated in mouse thyroid adenomas and adenocarcinomas. Thyroids at various stages of tumour development were analyzed by semi-quantitative RT-PCR and western blot. As previously described, neither FasL mRNA, nor FasL protein could be detected in normal thyroid tissue from NTG mice. In non pathological thyroid glands from gsp transgenic mice, FasL mRNA and protein were absent in all mice tested (see the above Table 1, and see Fig. 1A and 1B respectively) indicating that transgene expression by itself did not induce FasL molecule. By contrast, FasL mRNA and protein could be detected in hyperplastic thyroids and at higher level in thyroid adenomas and adenocarcinomas (Table 1 and Fig. 1A and 1B). The results showed that only diseased lobes were positive for FasL. Moreover, the soluble form of FasL was always detected in adenomas and was expressed stronger than the membrane-bound form of FasL. Those results indicate that thyrocytes acquire FasL expression during tumourigenesis. By contrast, RT-PCR and western blot analysis showed that Fas mRNA and protein were indifferently expressed both in non pathological and pathological thyroids (Table 1).

### Daxx and RIP are continuously expressed during tumour development whereas the adapter protein FADD is lost.

The modulation of the three adapter molecules for the Fas receptor was first investigated by semi-quantitative RT-PCR (Table 1 and Fig. 2A). FADD, Daxx and RIP mRNAs were detectable in thyroids from NTG and gsp transgenic mice, and mRNAs level of expression was not significantly modified during the course of tumour growth. Western blot analysis showed that high level of FADD protein was detected in all non pathological (3/3) and hyperplastic (4/4) glands of gsp mice. Strikingly, FADD protein expression was very weak (2/5) or completely lost (3/5) in adenomas and adenocarcinomas (Table 1 and Fig. 2B panel 1). Immunohistochemistry confirmed those results and showed that FADD protein expression was limited to restricted areas or completely lost in adenomas and adenocarcinomas. By contrast, western blot (Table 1 and Fig. 2B) and immunohistochemistry analysis confirmed that RIP and an approximately 70 kDa form of Daxx proteins were both expressed at all stages of tumour development in gsp transgenic mice. Daxx protein expression was unmodified or moderately decreased in adenomas/adenocarcinomas (Fig. 2B panel 2). Whatever the tumour status of the gland, the 120 kDa form of Daxx could not be detected even in the nucleus . Compared to non pathological thyroid gland, RIP protein expression was slightly increased in adenomas (Fig. 2B panel 3).

### Daxx mediated signal is a main Fas signalling pathway in absence of FADD.

The above results showed that both Daxx and RIP, but not FADD protein, were expressed in thyroid adenoma/adenocarcinoma. The next step was to study the downstream effector proteins of the Daxx and RIP signalling pathways. Western blot analysis of ASK1 protein, the intermediate between the adapter Daxx and the kinases pathway, showed that this molecule was expressed in thyroids of gsp transgenic mice at all stages of tumour development, and that ASK1 expression strongly increased during tumour growth (Table 1). On the other hand, RIP adapter can induce apoptosis due to caspase 2 activation. Western blot analysis showed that Pro-caspase 2 long (48 kDa) was expressed at high level in all tested thyroids, independently of the tumour status of the gland. By contrast, inhibitory forms of caspase 2 (Pro-caspase 2 short (35 kDa) and/or caspase 2 long-Prodomain (15 kDa) were only expressed in tumour specimen. Although Pro-caspase 2 short (35 kDa) was barely expressed in adenomas samples, high level of caspase 2 long-Pro (15 kDa) was detected (Table 1). The inhibitory 15 kDa form of caspase 2 was detected in 1 out of 2 hyperplastic gland. Those results suggested that although RIP-caspase 2 pathway could be induced during tumour development of gsp transgenic mice, it was not the main Fas-signalling pathway in this situation. Indeed, all adenomas expressed the 48 kDa apoptotic form of caspase 2, but expressed an inhibitory (35 kDa and/or 15 kDa) form of caspase 2 too. Thus, in absence of FADD, Fas signalling seems to act mainly through a Daxx-ASK1 pathway.

### Inhibition of the kinases pathway during tumour development.

We further investigated by western blot analysis the two main kinase signalling pathways downstream of ASK1. Total MKK3 protein was expressed in thyroid of gsp transgenic mice. However, expression strongly decreased during tumour development (Table 1). The ser189 phospho-MKK3 and ser207 phospho-MKK6 proteins, which are active forms of the kinases, are both recognized indifferently by the antibody we used. Phosphorylated proteins were strongly detected in gsp thyroids independently of the tumour status of the gland (Table 1). Those results demonstrated that in hyperplastic thyroids and adenomas, only MKK6 protein is activated as total MKK3 was poorly expressed. The average expression of total p38-MAPK, the kinase downstream of MKK3/MKK6, was identical in the different groups (Fig. 3 upper panel). By contrast, expression of the p38-phosphorylated active form was strongly decreased during tumour development (Fig. 3 lower panel). Indeed, high level of thr180/tyr182 phospho-p38-MAPK protein was detected in all non pathological thyroids of gsp transgenic mice, whereas the expression of this active form of the protein was low in hyperplastic glands, and very weak or absent in adenomas. Moreover, decrease or absence of p38-MAPK activation correlated with decrease or absence of total MKK3 expression . The other kinases pathway activated by ASK1 implicates SEK1/MKK4 and SAPK/JNK molecules. Total SEK1 protein was detected in thyroids of gsp mice at all stages of tumour development, but level of expression was higher in adenomas than in non pathological and hyperplastic gland. By contrast, the thr261 phospho-SEK1 active form was expressed stronger in non pathological thyroids than in hyperplastic glands or adenomas (Table 1). Although total and thr183/tyr185 phospho-SAPK/JNK proteins level of expression was similar in all groups of mice, apparent molecular weight of phosphorylated proteins was higher in non pathological thyroids compared to disease glands (Table 1). These results suggested that SAPK/JNK proteins are phosphorylated at both thr183 and tyr185 sites in non pathological thyroids, but that hyperplastic glands and adenomas are mono-phosphorylated due to a lack of phospho-SEK1 dependent thr183 phosphorylation. As dual phosphorylation of SAPK/JNK is necessary for kinase activity, these results showed that the ASK1/SEK1/JNK pathway, as well as the ASK1/MKK3/p38-MAPK signalling pathway, are strongly inhibited during tumour development.

### In vitro cultured thyrocytes did not expressed FADD protein.

In the course of our study, we performed *in vitro* cultures of normal non transgenic TFC and investigated the presence of molecules of the Fas signalling pathway by semi-quantitative RT-PCR and western blot analysis. Surprisingly, although FADD mRNA was expressed (Fig. 4A), FADD protein was undetectable in cultured thyrocytes (Fig. 4B). Further investigations showed that loss of FADD protein expression was induced by dissociation of thyroid tissue with collagenase H (Fig. 4B) and that FADD protein could not be detected thereafter, even following 10 days of culture . From the above results, it was important to know whether lack of FADD protein was an intrinsic property of cultured thyroid cell or a generalized FADD protein expression regulation mechanism. Hepatocytes were chosen to compare with thyrocytes because they are epithelial cells and liver tissue, like thyroid tissue, needs collagenase H treatment before culture. Western blot analysis was performed on *ex vivo* liver, collagenase H treated and *in vitro* cultured hepatocytes. *Ex vivo* liver, like *ex vivo* thyroid, expressed FADD protein (Fig. 4C). However, in contrast to TFC, western blotting of collagenase H treated and *in vitro* cultured hepatocytes showed presence of FADD protein (Fig. 4C). These results suggested that loss of FADD protein expression was restricted to thyroid epithelial cells and that this *in vitro* culture system could be used to elucidate the consequences of FADD protein expression regulation by thyrocyte.

### Fas signalling in absence of FADD increases TFC growth.

To evaluate the biological consequences of the Fas/FasL interaction in absence of FADD protein, thyroid cells were cultured with or without anti-Fas antibody and proliferation of thyrocytes was determined every day until confluence. Figure 5 showed that after 24h of treatment, there was no significant difference between proliferation of control and anti-Fas treated TFC. Thereafter, anti-Fas antibody accelerated growth of TFC compared to not treated TFC. Moreover, anti-Fas antibody-induced growth increase significantly after 48h, 72h and 96h of treatment (37% (p<0.02), 49% (p<0.05) and 61% (p<0.001) respectively). No more difference between control and anti-Fas treated TFC growth could be detected when cultures reached confluence (after 120h of treatment).

### Discussion

The expression of a large array of molecules involved in the Fas/FasL pathway during the *in vivo* transformation process of murine thyroid follicular cells (TFC) was investigated by using multiple complementary techniques. We showed that during the transformation process from non pathological TFC to hyperplastic nodules and adenoma or adenocarcinoma, 1) FasL expression is gained; 2) FADD protein but not mRNA is lost; 3) Fas signalling in absence of FADD results in a Daxx- but not RIP-mediated signal; 4) disappearance of FADD protein was correlated with inactivated status of the p38-MAPK; 5) Fas signalling in absence of FADD accelerated TFC growth. This is the first instance in which spontaneous FADD protein disappearance has been associated with pathological consequence.
In mice, transgenic expression of a mutant form of Gsα subunit directed to TFC induces adenomas or adenocarcinomas. In these mice, pathological lesions start around 8 months of age, and thyroid tumour develops thereafter. To elucidate the pathological event that could explain such tumour development, we focus on the Fas/FasL expression as well as Fas signalling molecules. Indeed, FasL expression could have a double advantage for the cells. First, it was reported that FasL expression on tumour cells permits elimination of infiltrating lymphocytes that express the Fas receptor (counter-attack model). Second, a new concept has recently emerged showing that Fas/FasL interactions not only promote cell death of Fas⁺ target cells, but can also stimulate proliferation of the same Fas⁺ cells. Thus, FasL expression on tumour cells will confer a double advantage to these cells by inducing apoptosis of invading cytotoxic lymphocytes, and by stimulating their own proliferation through Fas/FasL interaction. Nevertheless, regulatory mechanisms that take place in tumour cells to block the death signal, whereas maintaining the transduction of a proliferative signal mediated by Fas, are poorly understand.
The fact that gain of FasL expression during tumour development was concomitant with loss of FADD protein suggested that absence of FADD may allow tumour cells to escape a suicide death that could be induced by Fas/FasL co-expression.
In gsp transgenic mice, our results showed that FADD protein might have a tumour suppressive function. As a matter of fact, Fas signalling in TFC in absence of FADD appears to promote cell growth instead of apoptosis. Indeed, in a culture model of thyrocytes, our results showed that stimulation of TFC by anti-Fas antibody, in absence of FADD, resulted in a higher proliferation of these cells compared to non stimulated cells. Furthermore, those results were confirmed by the higher proliferative rate of TFC expressing FasL, isolated from thyroid specific FasL transgenic mice, compared to non transgenic cultured thyrocytes .
Our results show that gain of FasL, with concomitant loss of FADD expression, could confer multiple advantages to thyroid tumour cells: 1) high resistance to Fas-mediated cell death, 2) induction of proliferation via Fas signalling, 3) potential elimination of infiltrating lymphocytes.
FADD is a common signalling molecule to different death receptors. Stimulation of TNFR1 (tumour necrosis factor (TNF) receptor 1) with TNF leads to recruitment of the adapter TRADD (TNFR-associated death domain) which can in turn bind FADD and TRAF2 (TNFR-associated factor 2). As FADD protein is not expressed in thyroid adenomas and adenocarcinomas, TNF stimulation would result only in a TRAF2 dependent transducing signal. Furthermore, FADD-deficient embryonic fibroblasts were shown to be resistant to apoptosis induced by TNFR1, suggesting that FADD is essential to TNF-mediated cell death. Indeed, in our *in vitro* thyrocytes culture model, we found that FADD deficient TFC were resistant to TNFα-mediated cell death , supporting the idea that gsp transgenic mice acquired Fas- but also TNFR1-induced apoptosis resistance. FADD is also necessary to DR3 (death receptor 3) induced apoptosis, as DR3 resembles TNFR1 and activates similar signalling molecules upon APO3L stimulation. TRAIL (TNF-related apoptosis-inducing ligand) is implicated in tumour cells elimination. In these cells, two death receptors, DR4 (TRAIL-R1) and DR5 (TRAIL-R2), can transduce apoptotic signals. Requirement of FADD for TRAIL-mediated cell death was demonstrated using FADD-deficient embryonic fibroblasts stably transfected with DR4 or DR5). Moreover, primary thyrocytes, as well as thyroid papillary carcinoma-derived cell line KAT5 were shown to express DR4 and DR5 but to resist TRAIL-induced apoptosis. All these data showed that apoptosis induced by Fas, TNFR1, DR3, DR4 and DR5 needs FADD molecule, implicating that all these death pathways may be blocked in gsp transgenic mice developing adenomas or adenocarcinomas. Thus, FADD deficiency would confer multiple resistance against death receptors-induced apoptosis to tumour cells, and would allow opening of multiple proliferation pathways.
Signal transmission through Fas can operate through three different adaptators. In the absence of FADD, only Daxx and RIP proteins can transduce a Fas-mediated signal. However, we have showed here that RIP signalling was not elicited in FADD deficient thyroids, and that ASK1 intermediate expression strongly increased in adenomas/adenocarcinomas, but without consequent p38-MAPK or JNK pathways activation. As Daxx still is a poorly known molecule, we can make three hypothesis concerning the mechanism of Fas-induced proliferation of TFC in absence of FADD: 1) Daxx signalling is directly responsible for cell proliferation, by regulating the balance between MKK3, MKK6 and SEK1/MKK4 activation, 2) Daxx is an adapter protein leading to a still unknown cell growth transducing pathway, 3) Daxx is not implicated in this phenomenon and a still unknown adapter for the Fas receptor transduces a proliferative signal (see scheme on Fig. 6).
Activation of proliferation in response to Fas engagement has been previously described in different cell types. In normal T lymphocytes population, consequence of Fas ligation depends of previous antigenic stimulation. Indeed, following Fas ligation, memory CD4⁺ T cells are induced to proliferate whereas naive CD4⁺ T cells undergo apoptosis both *in vitro* and *in vivo.* In human dermal fibroblasts, induction of apoptosis or proliferation depends from the level of Fas receptor expression. Although anti-Fas antibody stimulates proliferation of low Fas expressing fibroblasts, anti-Fas treatment triggers apoptosis of high Fas expressing fibroblasts that mimic inflamed skin. More surprisingly, in liver, which is a Fas-mediated apoptosis highly sensitive cell, Fas engagement can accelerate organ regeneration after partial hepatectomy in vivo, instead of inducing fulminant hepatitis. All these data demonstrated that signal through Fas can result either in apoptosis or in proliferation, depending of the type of cells and the environmental conditions. In tumour cells, anti-Fas promoted cell growth has been reported in a few cases and concerned hematologic well as non hematologic tumours. In human thyroid carcinoma cells, it was reported that both Fas and FasL were expressed, but that cross-linking of Fas failed to induce recruitment and activation of caspase 8. It would be interesting to test the presence of FADD protein in those cells, as lack of this molecule could explain Fas-mediated cell death resistance, and strong proliferation of tumour cells.
In a more general approach, FADD expression may be tested in all types of cancer expressing Fas and FasL molecules, but resistant to Fas-induced apoptosis.
In eukaryotic cells, translation of mRNAs is mainly due to a cap-dependent mechanism, implicating proteins of the eukaryotic initiation factor 4 (eIF4) family. The multiproteic complex eIF4F can recognize the m⁷GpppN cap structure and initiate the ribosome scanning of the mRNA. On the other hand, few mRNAs are translated by a cap-independent mechanism. In this case, ribosome entry occurs at a specific region in the 5' end of the mRNA called internal ribosome entry segment (IRES). Such mRNAs possessing IRES have been described in mammalians and are implicating in control of cell growth. Among them, c-myc mRNA which can be translated by cap-dependent or cap-independent mechanisms. It was demonstrated that during apoptosis of HeLa cells induced by TRAIL, c-myc mRNA translation occurred via the IRES, and that the p38-MAPK signalling pathway was implicated in this cap-independent translation. Strikingly, the thr180/tyr182 phospho-p38-MAPK pattern of expression was very similar to that of FADD (Fig.2 B and 3). Indeed, although both phospho-p38-MAPK and FADD proteins were expressed in non pathological gsp thyroids, both proteins were absent in adenomas/adenocarcinomas. Importantly, no cap site was found in FADD mRNA. This suggested that, like c-myc, FADD mRNA regulation of cap-independent translation could occur via activation of p38-MAPK pathway. In thyroid tumour cells, inhibition of p38-MAPK signalling would lead to blockade of cap-independent mRNA translation, including FADD and c-myc which are both involved in apoptosis. Absence of expression of these proteins, and particularly FADD, can contribute to cancer progression by deviating FasL signalling from a death to a proliferative pathway.
Our data suggest a new role for the Fas/FasL pathway in the etiology of thyroid tumour. In absence of FADD protein, but in presence of Fas and FasL molecules, thyrocytes would have numerous advantages including high resistance to Fas and other death receptors-mediated apoptosis, stimulation of their own proliferation through Fas/FasL interaction, and capacity to counter-attack the infiltrating lymphocytes allowing immune escape.
Understanding how FADD protein expression is lost will permit to envisage new therapeutic approaches. Indeed, restoration of FADD function in FasL⁺ thyrocytes would induce higher Fas-mediated cell death susceptibility, and inhibit Fas-induced proliferation of these cells, leading to tumour regression. Moreover, the present results may reflect a feature which is common to most cancers wherein FasL expression and/or high resistance to apoptosis-inducing drugs have been reported.

### EXAMPLE 2: expression of FADD is also lost in blood samples of myeloid leukaemia human chemo-resistant patients.

Blood samples from human patients for which a diagnosis of acute or chronic myeloid leukaemia (AML or CML) have been established were tested for FADD expression. All blood samples tested contained at least 80% of tumour cells. The results thus obtained are as follows:

**Table 2:**

| **human AML** | | | | |
|---|---|---|---|---|
| Patient's reference name | Disease | **Reaction totentative therapy** at the date of sampling | Sampling date dd.mm.yy (dose) | **FADD** |
| 1A | AML | In remission | 10.08.01 | +++ |
| 3L | AML 5 | S | 25.09.98 | ++ |
| 3S | AML 2 | R | 04.04.00 (10.10⁶) | + |
| | | | | + |
| 4V | AML | R | 27.04.98 | +/- |
| 5A | AML 4 | R | 04.07.01 | +/- |
| 2M | AML | R | 27.04.00 (25.10⁶) | + |
| | | | | ++ |
| 8R | AML 5 | R | 17.10.01 (49.10⁶) | + |
| | | | | +/- |
| 9N | AML0 | *nd* | 10.11.97 (20.10⁶) | ++ |
| | | | | + |
| 6A | AML | *nd* | 15.10.97 | +++ |
| 5M | AML | *nd* | 10.03.00 (40.10⁶) | + |
| | | | | ++ |
| **S** = chemo-sensitive to anti-tumour chemotherapy | | | | |
| **R**= chemo-resistant to anti-tumour chemotherapy | | | | |
| **nd** = not yet determined | | | | |
| **Expression of FADD,** as assessed by the intensity of the 28-30 kDa band on Western blots (blind test analysis): - **- or** +/-: null or very low, - +: low, - ++: medium, - +++: high. | | | | |

**Table 3:**

| **human CML** | | | | |
|---|---|---|---|---|
| Patient's reference name | Disease | **Reaction to tentative therapy** at the date of sampling | Sampling date dd.mm.yy (dose) | **FADD** |
| 1D | CML | S | 31.05.00 (6.10⁶) | +++ |
| | | | | +++ |
| 1C | CML | R in relapse | 18.05.01 (20.10⁶) | + or +/- |
| 2L | CML | R, relapse after graft, but decrease in bcr-abl mRNAs after immunosuppressive treatment has been stopped | 29.03.00 (40.10⁶) | - |
| | | | | +/- |
| 30 | CML | R | 05.02.96 (7.10⁶) | - |
| | | | | - ? |
| 4E | CML | R | 21.09.98 (30.10⁶) | - |
| | | | | - |
| 6R | CML | R relapse after graft | 14.05.98 (17.10⁶) | - |
| | | | | - |
| 5R | CML | IFNα resistant No relapse after graft | 30.03.98 (19.5.10⁶) | - |
| | | | | +/- ? |
| 8A | CML | Grafted in Sept. 2000 + immunosuppressive treatment: | 11.05.01 (10.10⁶) graft's lymphocytes | +/- |
| | | | | - |
| Anti-tumour treatment = IFNα tentative therapy, followed in case of resistance, by bone marrow allograft (tentative GVL effect via Fas) STI: drug inducing apoptosis by blockade of the bcr-abl pathway | | | | |

## Claims

1. Use of at least one protein selected from the group consisting of FADD proteins and phosphorylated p38-MAPK, as a marker for absence *of in vivo* tumour.

2. A method to determine whether a suspected tumour has developed or spread or is in development or spreading in an animal, or whether such a tumour is absent or has regressed near to remission or recovery, **characterised in that** it comprises the following steps:
- a biological sample collected from said animal as representative of said suspected tumour is provided,
- at least one content selected from the group consisting of the content in FADD proteins, and the content in phosphorylated p38-MAPK, is measured in said sample, and
wherein said tumour is determined as having developed or spread or as being in development or in spreading, when said measured content(s) either is(are) not significantly different from zero, and/or is(are) significantly inferior to the standard normal (respective) content(s) which is(are) observed in a non-tumour but otherwise equivalent sample, and
wherein said tumour is determined as absent or as in regression near to remission or recovery, when said measured content(s) is(are) significantly different from zero and either not significantly different from or significantly superior to said standard (respective) normal content(s),
provided that said standard (respective) normal content(s) is(are) significantly different from zero.

3. A method to determine whether a tumour is in a phase of development or of spreading in an animal, whether it is in a phase of stabilization, or whether it is in a phase of regression, remission or recovery, by comparison with a previously-determined tumour status, **characterised in that** it comprises the following steps:
- a first biological sample which is representative of said tumour is provided,
- at least one content selected from the group consisting of the content in FADD proteins, and the content in phosphorylated p38-MAPK, is measured in said first sample, this(these) measured content(s) being referred to as the measured first content(s),
- a second representative biological sample collected at a time which is later than the collection time of said first sample is provided,
- the content(s) which has(have) been measured in said first sample are then measured in said second sample, this(these) measured content(s) being referred to as the measured second content(s), and
wherein said tumour is determined as being in a phase of development or of spreading, when said measured second content(s) is(are) significantly inferior to said measured (respective) first content(s),
wherein said tumour is determined as being in a phase of stabilization, when said measured second content(s) is(are) not significantly different from said measured (respective) first content(s),
wherein said tumour is determined as being in a phase of regression, remission or recovery, when said measured second content(s) is(are) significantly superior to said measured (respective) first content(s),
provided that the standard normal (respective) content(s) which is(are) observed in a non-tumour but otherwise equivalent sample is(are) significantly different from zero.

4. A method according to claim 3, **characterised in that** said animal is subjected to a tentative anti-tumour therapy after the collection of said first sample, but before the collection of said second samples.

5. A method to determine whether a treatment is, or will be, efficient to treat or to arrest the growth of a tumour which is present or in development or in spreading in an animal, **characterised in that** it comprises the following steps:
- a biological sample which has been collected from said animal or from a representative biological model thereof wherein a comparable tumour is present or in development or in spreading, as representative of said tumour during the course, or at the end of said treatment is provided,
- at least one content selected from the group consisting of the content in FADD proteins and the content in phosphorylated p38-MAPK is measured in said sample, and
wherein said treatment is determined as efficient for said animal, when said measured content(s) is(are) not significantly different from the standard (respective) normal content(s) in a non-tumour but otherwise equivalent biological sample,
provided that said standard (respective) normal content(s) is(are) significantly different from zero.

6. A method to determine whether a treatment is, or will be, efficient against a tumour which is present but not developing or spreading in animal, or whether it is, or will be, inefficient, **characterised in that** it comprises the following steps:
- a representative biological sample which has been collected from said animal or from a representative biological model thereof wherein a comparable tumour is present, as representative of said tumour, during the course or at the end of said treatment is provided,
- at least one content selected from the group consisting of the content in FADD proteins and the content in phosphorylated p38-MAPK, is measured in said sample,
wherein said treatment is determined as efficient, when said measured content(s) is(are) significantly superior to the (respective) control content(s) which is(are) present in the absence of said treatment,
wherein said treatment is determined as inefficient, when said measured content(s) is(are) either significantly inferior to, or not significantly different from said (respective) control content(s),
provided that the standard normal (respective) content(s) which is(are) observed in a non-tumour but otherwise equivalent sample is(are) significantly different from zero.

7. A method to determine whether a treatment is or will be efficient against a tumour which is in a phase of development or of spreading in an animal, or whether it is, or will be, inefficient, **characterised in that** it comprises the following steps:
- a representative biological sample which has been collected from said animal or from a representative biological model thereof wherein a comparable tumour is in development or spreading as representative of said tumour, during the course or at the end of said treatment is provided,
- at least one content selected from the group consisting of the content in FADD proteins and the content in phosphorylated p38-MAPK, is measured in said sample,
wherein said treatment is determined as efficient, when said measured content(s) is(are) either significantly superior to the (respective) control content(s) which is(are) present in the absence of said treatment,
wherein said treatment is determined as inefficient, when said measured content(s) is(are) significantly inferior to said (respective) control content(s),
provided that the standard normal (respective) content(s) which is(are) observed in a non-tumour but otherwise equivalent sample is(are) significantly different from zero.

8. Kit for determining whether there is or not a tumour in an animal, and/or whether an anti-tumour treatment is, or will be, efficient or not in an animal, **characterised in that** it comprises:
- at least one compound which is capable of specifically binding to FADD proteins and/or to phosphorylated p38-MAPK, or at least one biological unit which is capable of producing such a compound, this at least one compound or biological unit being possibly contained in a physiological buffer, and/or possibly bound to a solid support, and
- appropriate instructions for using FADD proteins and/or phosphorylated p38-MAPK as marker(s) of the absence of an *in vivo* tumour.

9. Use of a compound which is capable of specifically binding to FADD proteins and/or to phosphorylated p38-MAPK, or of a biological unit which is capable of producing such a compound, for determining on a representative biological sample, whether there is or not a tumour in an animal, and/or whether an anti-tumour treatment is, or will be, efficient or not in an animal.

10. Method to produce a product useful for determining whether there is or not a tumour in an animal, and/or whether an anti-tumour treatment is, or will be, efficient or not in an animal, **characterised in that** it comprises the step of producing said product by production of a compound which specifically binds to FADD and/or to phosphorylated p38-MAPK, possibly followed by binding a detection label to this anti-FADD and/or anti-phosphorylated p38-MAPK specific compound.

11. A method to assess whether a compound has a high or a low probability of having a tumour-inducing activity in a tumour-free animal, **characterized in that** it comprises the following steps:
- said compound is placed into contact with said tumour-free animal, or with a tumour-free representative biological model thereof,
- at least one content selected from the group consisting of the content in FADD proteins and the content in phosphorylated p38-MAPK, is measured in said animal or model, after said contact with said compound, and
wherein said compound is determined as having a high probability of having a tumour-inducing activity in said animal, when said measured content(s) is(are) significantly inferior to the standard (respective) normal content(s) which is(are) observed in the absence of contact with said compound,
wherein said compound is determined as having a low probability of having a tumour-inducing activity in said animal, when said measured content(s) is(are) not significantly inferior to said standard (respective) normal content(s),
provided that said standard (respective) normal content(s) is(are) significantly different from zero.

12. Kit to assess whether a compound has a high or a low probability of having a tumour-inducing activity in an animal, **characterized in that** it comprises at least one anti-FADD and/or anti-phospho-p38-MAPK specific compound, and possibly a tumour-free biological model.

13. A method to determine whether a compound can have an anti-tumour or anti-mitotic activity in an animal, **characterised in that** it comprises the following steps:
- non-tumour thyroid follicular cells (TFC) are placed for *in vitro* growth under conditions of substrate composition, of temperature and of time which enable the adhesion and confluence of said TFC,
- said compound is placed in contact with said culture,
- at least one content selected from the group consisting of the content in FADD proteins and the content in phosphorylated p38-MAPK is measured in said TFC after said contact with said compound, and
wherein said compound is determined as having an anti-tumour or anti-mitotic activity, when the measured content(s) is(are) either significantly superior to the (respective) content(s) which is(are) observed in a comparable TFC but in the absence of contact with said compound, or not significantly different from the (respective) content(s) which is(are) measured in *ex vivo* non-tumour TFC.

14. The method according to claim 13, **characterised in that** said non-tumour TFC are submitted to a collagenase H treatment before being placed for *in vitro* growth.

15. Kit to determine in the absence of any tumour-inducing agent whether a compound can have an anti-tumour or anti-mitotic activity in an animal, **characterised in that** it comprises:
- non-tumour thyroid follicular cells (TFC), or a lyophilisate thereof, and
- at least one anti-FADD and/or anti-phospho-p38-MAPK compound.

16. Use of a non-tumour thyroid follicular cell (TFC) as an *in vitro* model to determine in the absence of any tumour-inducing agent whether a compound has an anti-tumour or anti-mitotic activity.
